(19) Europäisches Patentamt
European Patent Office
Office européen des brevets

(11) Veröffentlichungsnummer: **0 197 294 B1**

(12) # EUROPÄISCHE PATENTSCHRIFT

(45) Veröffentlichungstag der Patentschrift: **03.04.91**

(51) Int. Cl.⁵: **C07D 501/20**, C07D 501/59, C07D 499/68, C07D 499/70, A61K 31/545, A61K 31/45, C07D 499/00

(21) Anmeldenummer: **86102770.4**

(22) Anmeldetag: **03.03.86**

Die Akte enthält technische Angaben, die nach dem Eingang der Anmeldung eingereicht wurden und die nicht in dieser Patentschrift enthalten sind.

(54) **Beta-Lactam-Antibiotika, Verfahren zur Herstellung und ihre Verwendung als Arzneimittel.**

(30) Priorität: **16.03.85 DE 3509618**

(43) Veröffentlichungstag der Anmeldung:
**15.10.86 Patentblatt 86/42**

(45) Bekanntmachung des Hinweises auf die Patenterteilung:
**03.04.91 Patentblatt 91/14**

(84) Benannte Vertragsstaaten:
**AT BE CH DE FR GB IT LI NL SE**

(56) Entgegenhaltungen:
**EP-A- 0 122 158**
**FR-A- 2 418 232**
**FR-A- 2 418 234**
**GB-A- 2 015 512**

(73) Patentinhaber: **BAYER AG**

**W-5090 Leverkusen 1 Bayerwerk(DE)**

(72) Erfinder: **Schmidt, Gunter, Dr.**
**Pahlkestrasse 63**
**W-5600 Wuppertal 1(DE)**
Erfinder: **Zeiler, Hans-Joachim, Dr.**
**Am Rohm 86**
**W-5600 Wuppertal 1(DE)**
Erfinder: **Metzger, Karl Georg, Dr.**
**Pahlkestrasse 75**
**W-5600 Wuppertal 1(DE)**

**EP 0 197 294 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft β-Lactam-Antibiotika, Verfahren zu ihrer Herstellung sowie ihre Verwendung in Arzneimitteln, insbesondere als oral wirksame Antibiotika

Es sind bereits verschiedene α-Aminoacyl-cephalosporin-Antibiotika bekannt, so z. B. Cefalexin (GB-PS 1 174 335) oder Cefaclor (DE-OS 2 408 698 und DE-OS 2 728 578).

Die Erfindung betrifft β-Lactamverbindungen der allgemeinen Formel (I)

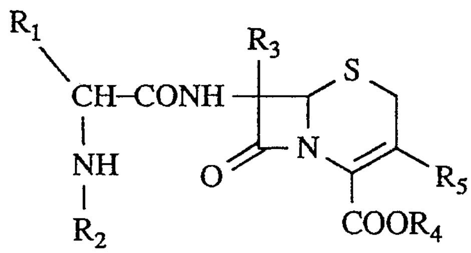

(I)

worin

$R_5$ für Wasserstoff, Halogen, Azido oder für geradkettiges, verzweigtes oder cyclisches, gesättigtes oder ungesättigtes Alkyl mit bis zu 6 C-Atomen steht, das gegebenenfalls substituiert ist durch Halogen, $OCONH_2$, $C_1$-$C_5$-Alkoxy, $C_2$-$C_7$-Acyloxy oder $C_1$-$C_5$-Alkylthio steht,

$R_1$ für einen Rest der Formel

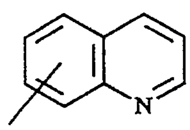 , 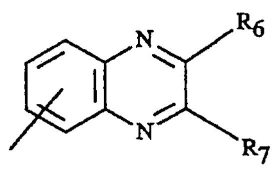 , 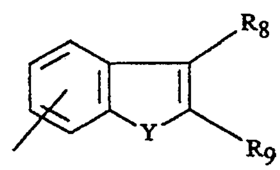

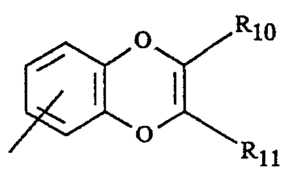 , 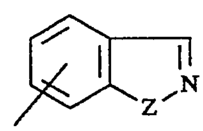 , 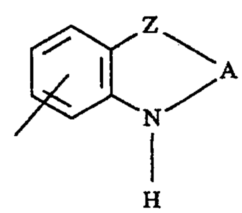

steht,

worin

$R_6$, $R_7$ gleich oder verschieden sind und für Wasserstoff,

für $C_6$-$C_{10}$-Aryl, für eine Aminogruppe oder für Hydroxy stehen,

$R_8$, $R_9$ gleich oder verschieden sind und für Wasserstoff,

für Hydroxy, für eine Aminogruppe, für

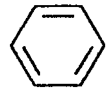 , 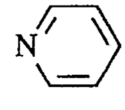 ,

für Alkyl mit bis zu 8 C-Atomen, das substituiert ist durch Phenyloxy,

für Phenyl, das substituiert ist durch Chlor oder Amino, oder

für Alkyl mit bis zu 12 C-Atomen stehen oder

$R_5$ und $R_9$ zusammen für die Gruppe

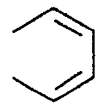

stehen,
A für die Gruppe

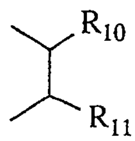 oder 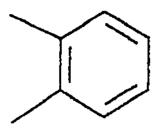

steht,
$R_{10}$, $R_{11}$ gleich oder verschieden sind und für Wasserstoff,
für Alkyl mit bis zu 12 C-Atomen,
Y für Sauerstoff oder -NH steht,
Z für Sauerstoff, Schwefel oder -NH steht,
und worin
$R_2$ für Wasserstoff oder eine der folgenden Aminoschutzgruppen,
tert.-Butoxycarbonyl (Boc), Carbobenzoxy (Cbz), 2-Methoxycarbonyl-1-methylvinyl, Trityl (Trt), Benzyl (Bzl), Benzyloxycarbonyl (Z), Formyl oder Chloracetyl,
$R_3$ für Wasserstoff, für Alkoxy, Alkylthio mit jeweils bis zu 5 C-Atomen, und
$R_4$ für Wasserstoff oder für eine der folgenden Carboxyschutzgruppen
tert.-Butyl, Decyl, 2,2,2-Trichlorethyl, Benzyl, 4-Methoxybenzyl, 4-Nitrobenzyl, Triphenylmethyl, Diphenylmethyl, Acetoxymethyl, Pivaloyloxymethyl, Allyl oder Trimethylsilyl
für Alkaliionen oder Ammoniumionen oder für einen Rest der Formel $-CH_2-O-CO-C(CH_3)_3$ oder $-CH(CH_3)-O-CO-O-C_2H_5$ steht.

Bevorzugte Verbindungen der Formel (I) sind solche, in denen $R_5$ Fluor, Chlor, Brom, Methoxy, Methylthio, Trifluormethyl, Methoxymethyl oder für gegebenenfalls durch Halogen oder $OCONH_2$ substituiertes Methyl, Vinyl, $-CH=CH-CH_3$, $-CH=CH-C_2H_5$ steht.

Die Verbindungen der Formel I können als freie Säuren. Ester, als innere Salze oder als nicht-toxische pharmazeutisch verträglichen Salze der sauren Carboxylgruppen, wie Natrium-, Kalium-, Magnesium-, Calcium-, Aluminiumund Ammoniumsalze und nicht-toxische substituierte Ammoniumsalze, mit Aminen wie Di-, Tri-niedrigalkylaminen, Procain, Dibenzylamin, N,N'-Dibenzylethylendiamin, N-Benzyl-β-phenyl-ethylamin, N-Methyl- und N-Ethylmorpholin, 1-Ephenamin, Dehydroabietylamin. N,N'-Bis-dehydroabietylethylendiamin, N-Niedrigalkylpiperidine und anderen Amine, die zur Bildung von Salzen von Penicillinen und Cephalosporinen verwendet werden können, vorliegen.

Wegen der Anwesenheit des mit * bezeichneten asymmetrischen Kohlenstoffatoms schließen die neuen β-Lactamantibiotika der Formel I die D-, L- und D,L-Formen ein. Bevorzugt sind die D-Formen der erfindungsgemäßen Verbindungen der allgemeinen Formel I.

Sowohl die Diastereomerengemische als auch die D-Form und L-Form der erfindungsgemäßen Verbindungen können zur Behandlung bakterieller Infektionskrankheiten eingesetzt werden.

Die Verbindungen der allgemeinen Formel I erhält man, wenn man Verbindungen der allgemeinen Formel IIa

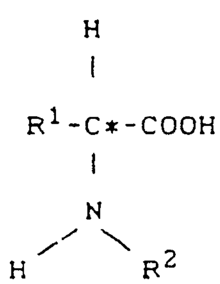

(IIa)

in welcher

$R^1$ die oben angegebene Bedeutung hat und in welcher

$R^2$ für eine in der $\beta$-Lactam-Chemie übliche Aminoschutzgruppe steht,

nach Aktivierung der Carboxylgruppe durch Uberführung in ein gemischtes Anhydrid, beispielsweise mit Pivaloylchlorid, Chlorameisensäureethyl oder isobutylester. nach Überführung in das Mesylat mittels Methansulfonsäurechlorid oder nach Überführung in einen aktivierten Ester, z.B. mit 1 = Hydroxybenztriazol und Dicyclohexylcarbodiimid,

mit Verbindungen der allgemeinen Formel III

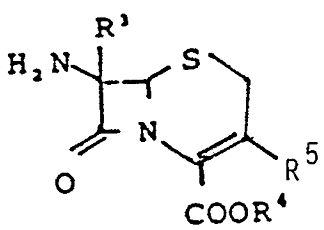

(III),

in welcher $R^3$, $R^4$ und X die oben angegebenen Bedeutung haben, zur Umsetzung bringt, dann gegebenenfalls Schutzgruppen abspaltet und die gewünschten Salze oder aus den Salzen die freien Säuren herstellt.

Für die Kupplung der Aminosäuren der Formel II an $\beta$-Lactame der Formel III kann eine große Zahl von aus der Cephalosporin-Chemie bekannten Methoden verwendet werden.

Es hat sich als vorteilhaft erwiesen. Aminosäuren der allgemeinen Formel II zu aktivieren und dann mit $\beta$-Lactamen der allgemeinen Formel III, die als Salze mit einem Amin in Losung gebracht wurden. zu kuppeln. Besonders vorteilhaft ist die Aktivierung mit Pivalinsäurechlorid (IV b) oder Sulfonsäurederivaten der Formel IVa zu Anhydriden der Formel Va und Vb

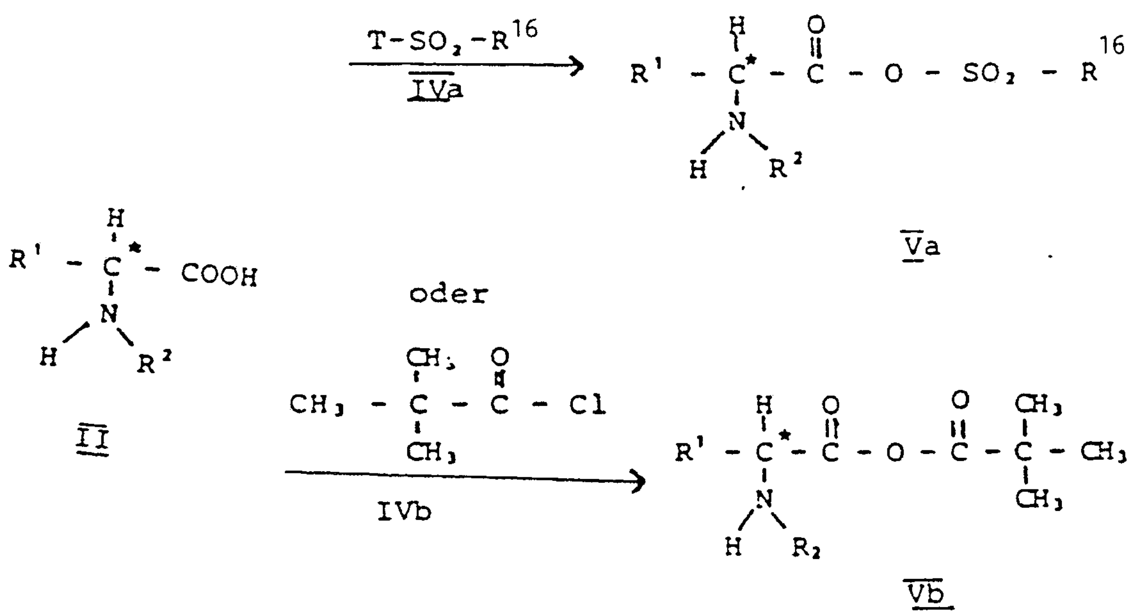


II

Va

Vb

in welchen

$R^1$ - die oben angegebene Bedeutung hat,

$R^2$ - für eine in der $\beta$-Lactam-Chemie übliche Aminoschutzgruppe steht,

T - für einen Rest $R^{16}SO_2O$ oder Halogen steht. und

$R^{16}$ - für Alkyl ($C_1$-$C_6$) steht. das gegebenenfalls substituiert ist durch Fluor. Chlor, Cyano, Phenyl, $C_1$-$C_4$-Alkyl, $C_1$-$C_4$-Alkoxy oder $C_1$-$C_4$-Alkylthio, oder

- für gegebenenfalls durch Fluor, Chlor, Brom. Cyano, $C_1$-$C_4$-Alkyl, $C_1$-$C_4$-Alkoxy, $C_1$-$C_4$-Alkylthio, $C_1$-$C_4$-Alkylcarbonyl, Nitro, Trifluormethyl oder Phenyl substituiertes Phenyl steht.

Wenn $R^{16}$ substituiert ist. sind vorzugsweise 1-3 Substituenten, vorzugsweise die genannten vorhanden. Ganz besonders bevorzugt stellt $R^{16}$ einen Methyl- oder p-Tolylrest dar.

Die gemischten Anhydride der Formeln Va und Vb werden hergestellt, indem man die Säuren der Formel II und 1-1,4-Äquivalente eines Amins in einem Lösungsmittel löst und mit 1 bis 1,2-Äquivalenten eines Sulfonsäurederivates der Formel IVa oder mit 1 bis 1,2-Äquivalenten Pivalinsäurechlorid der Formel IVb reagieren läßt.

Als Lösungsmittel eignen sich alle Solventien. die unter den Reaktionsbedingungen stabil sind, wie z.B. Diethylether, Tetrahydrofuran, Acetonitril, Aceton, Methylenchlorid, Chloroform, oder Dimethylformamid.

Als Amine eignen sich tertiäre Amine wie z.B. Triethylamin, Ethyldiisopropylamin oder Tributylamin, aber auch sterisch gehinderte sekundäre Amine, wie z.B. Diisopropylamin.

Die Umsetzungen können bei Temperaturen zwischen -80 °C und Raumtemperatur durchgeführt werden, bevorzugt zwischen -60 °C und 0 °C. Vorteilhaft wird die Aktivierung mit $Cl\text{-}SO_2\text{-}CH_3$ in Dimethylformamid bei -40 °C bis -60 °C innerhalb von 0,2 bis 24 Stunden, vorzugsweise 0,5 bis 5 Stunden, durchgeführt.

Zum Lösen der Verbindungen der Formel III können die bei der Herstellung der Verbindungen der Formel V genannten Lösungsmittel sowie als Base die dort genannten Amine verwendet werden.

Besonders vorteilhaft ist auch eine Aktivierung der Säuren der allgemeinen Formel II durch Überführung in einen aktivierten Ester mit beispielsweise N-Hydroxysuccinimid und Dicyclohexylcarbodiimid oder 1-Hydroxybenztriazol und Dicyclohexylcarbodiimid.

Als Lösungsmittel eignen sich alle Solventien, die sich auch für die Herstellung von Anhydriden der Formel V eignen.

Die Umsetzungen können bei Temperaturen zwischen -30 °C und +100 °C durchgeführt werden. Vorteilhaft wird mit 1-Hydroxybenztriazol und Dicyclohexylcarbodiimid in Dimethylformamid bei Raumtemperatur 2 bis 6 Stunden aktiviert, dann vom ausgefallenen Dicyclohexylharnstoff abgesaugt und mit einer Verbindung der Formel III in Form einer Lösung ihres Aminsalzes innerhalb von 2 bis 24 Stunden umgesetzt. Zum Lösen der Verbindungen der Formel III können die bei der Herstellung der Verbindungen der Formel V genannten Lösungsmittel sowie als Base die dort genannten Amine verwendet werden.

Literatur für Amino- und Carboxylschutz und Carboxy-Aktivierung: M. Bodanszky, Principles of Peptide Synthesis, Springer-Verlag, 1984. E. Gross, J. Meienhofer, The Peptides Vol. 2, Academic Press. 1980.

Die stereochemisch einheitlichen D-bzw. L-Formen der erfindungsgemäßen Verbindungen der Formel I erhält man wenn man die Diastereomerengemische beispielsweise an HPLC-Säulen von Merck, DuPont oder Whatmann trennt.

Andererseits erhält man die reine D- bzw. L-Form (bevorzugt die D-Form), wenn man bereits auf der Stufe der racemischen Aminosäure der Formel II eine chemische Racematspaltung, z.B. mit Dehydroabietylamin, Phenylethylamin und Camphersulfonsäure, oder eine RacematSpaltung z.B. über N-Acetyl-aminosäurederivate, z.B. mit Subtilisin, Penicillinacylase oder Schweinenierenacylase, vornimmt und anschließend die stereomisch einheitlichen D- bzw. L-Formen der Verbindungen der Formel II in angegebener Weise umsetzt.

Die Verbindungen der allgemeinen Formel II sind nur zum Teil bekannt. Die Verbindungen der Formel II können nach literaturbekannten Verfahren, wie es in Schema 1 dargestellt wird, synthetisiert werden, wobei die Verbindungen der Formel VI die wichtigsten Schüsselverbindungen für die neuen Aminosäuren der Formel II darstellen.

Schema 1

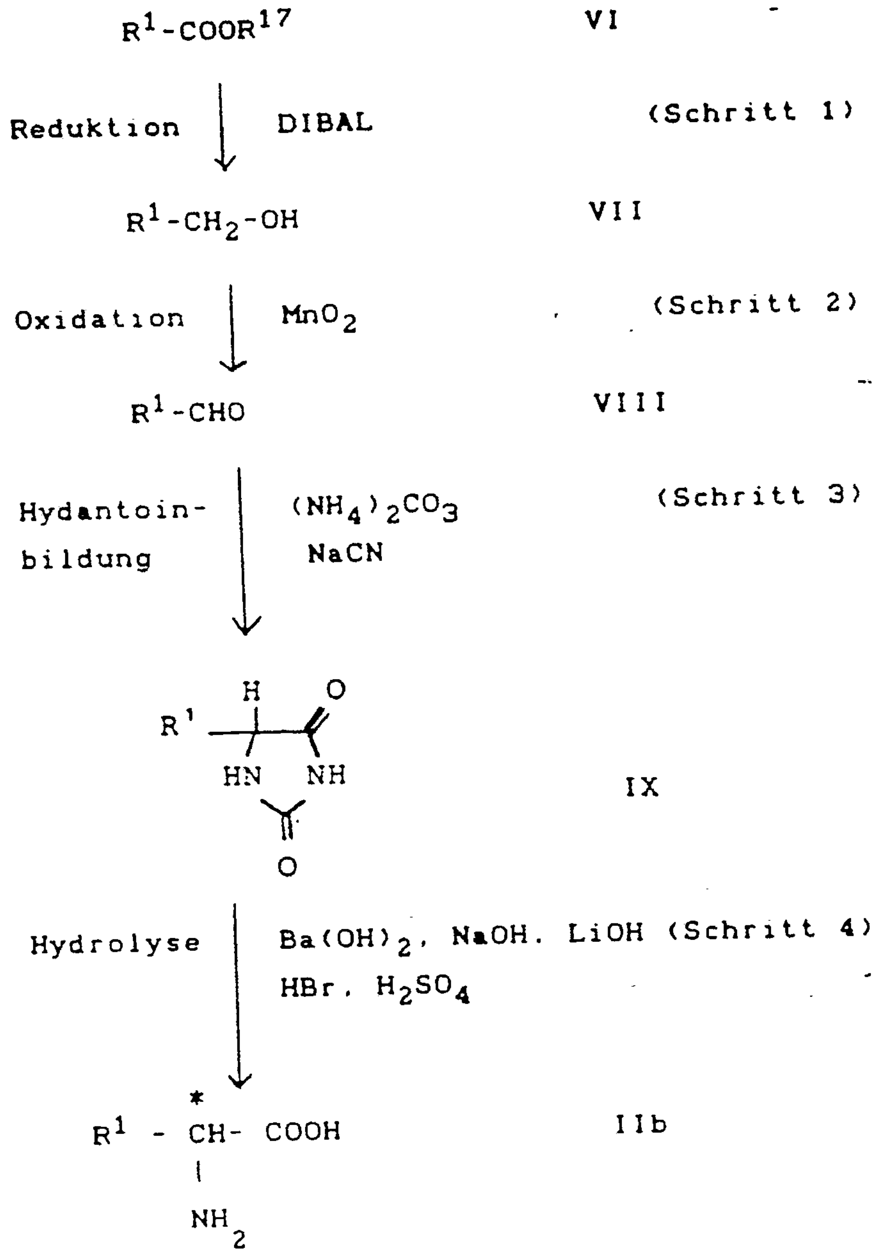


$R^1$ hat die oben angegebene Bedeutung,

$R^{17}$ steht für $C_1$-$C_4$-Alkyl.

Die Reduktion von Estern mit Diisobutylaluminiumhydrid (DIBAL) und Natrium-bis(2-methoxyethoxy)-aluminiumhydrid (Red-Al) zu Alkoholen (Schritt 1) ist in der Literatur beschrieben:

E. Winterfeld, Synthesis 1975 , 617; A.E.G. Miller et al., J. Org. Chem. 24 , 627 (1959).

Die Oxidation primärer Alkohole mit Mangan (IV)-oxid oder Pyridiniumchlorochromat in Aldehyde (Schritt 2) ist in der Literatur bekannt:

Methoden der Organischen Chemie, Houben-Weyl, Bd. 4/1 b; G. Piancatelli et al., Synthesis 1982 , 245.

Die neuen Aminosäuren der Formel IIb erhält man, wenn man die Aldehyde mit Natriumcyanid und Ammoniumcarbonat nach Literatur-bekannten Verfahren [E. Ware, Chemical, Reviews 46 , 403, (1950)] umsetzt (Schritt 3) und anschließend mit 10 %iger Natronlauge, 48 %iger Bromwasserstoffsäure, Bariumhydrox id- oder Lithiumhydroxidlosung hydrolysiert (Schritt 4).

Im folgenden wird beispielhaft die Darstellung einiger neuer Aminosäuren II bzw. deren Vorstufen beschrieben, wobei $R^1$-$R^{17}$ die oben angegebene Bedeutung haben:

1) Chinolyl- und Chinoxalyl-glycine

Ausgangsmaterial für die Synthese dieser benzokondensierten Ringsysteme sind Chinolin- und substituierte Chinoxalin-carbonsäurederivate. Substituierte Chinoxalincarbonsäurederivate werden beispielsweise nach folgendem Syntheseschema hergestellt:

Schema 2

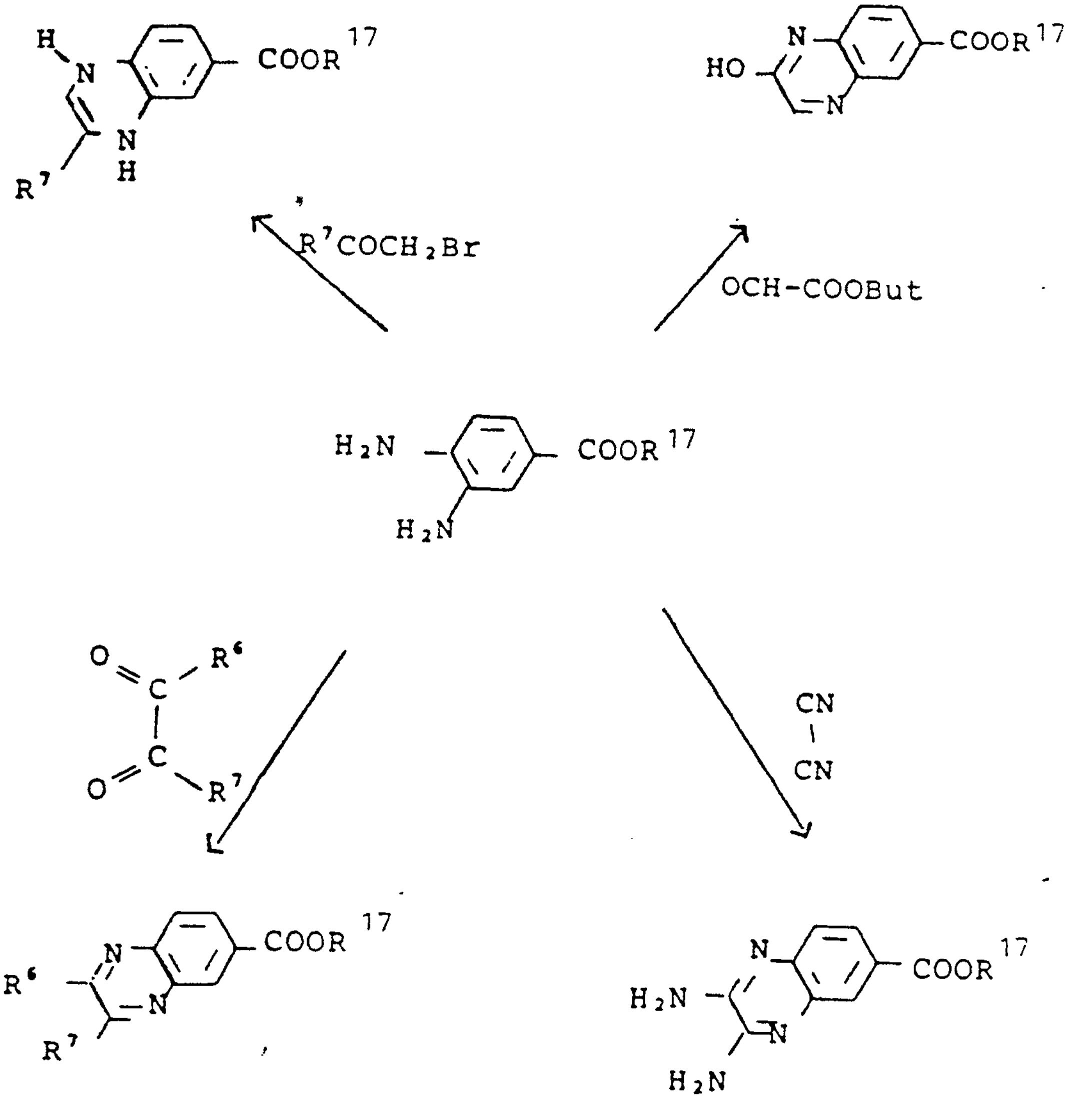


Literatur: G.W.H. Cheesman et al.. Quinoxaline Chemistry, Advances in Heterocyclic Chemistry, Vol. 22. 367, Academic Press (1978).

2) 1.4-Benzodioxinyl-glycine

Für die Synthese von substituierten 1.4-Benzodioxinyl-glycinen verwendet man als Ausgangsmaterial 3.4-Dihydroxybenzoesäurederivate. Die neuen 1.4-Benzodioxin-6-carbonsäureester werden beispielsweise nach folgendem Syntheseschema hergestellt:

Schema 3

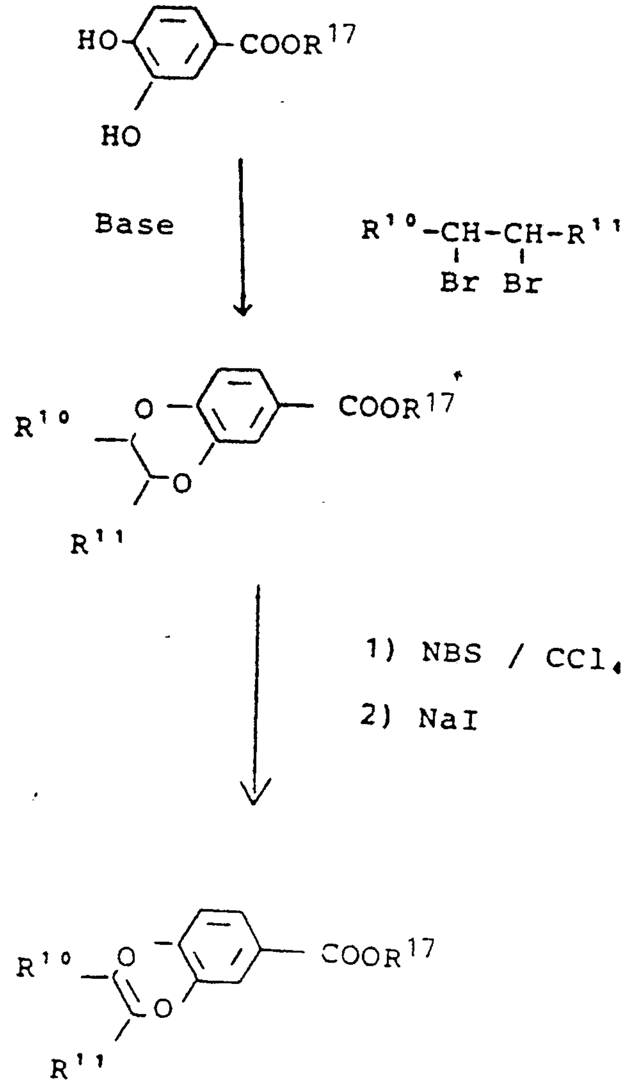


Literatur : W. Adam et al., Synthesis 1982 . 322

3) 1.4-Benzoxazinyl-. 1.4-Benzthiazinyl- und 1.2.3.4-Tetrahydrochinoxalylglycine

Für die Synthese dieser anellierten Phenylglycine verwendet man als Ausgangsmaterial 3-Hydroxy-4-aminobenzoesäure. 3-Amino-4-hydroxybenzoesäure. 3-Amino-4-mercaptobenzoesäure und 3.4-Diaminobenzoesäure in Form ihrer Ester. Die neuen benzo-kondensierten Carbonsäureester werden beispielsweise nach folgendem Syntheseschema hergestellt:

Schema 4

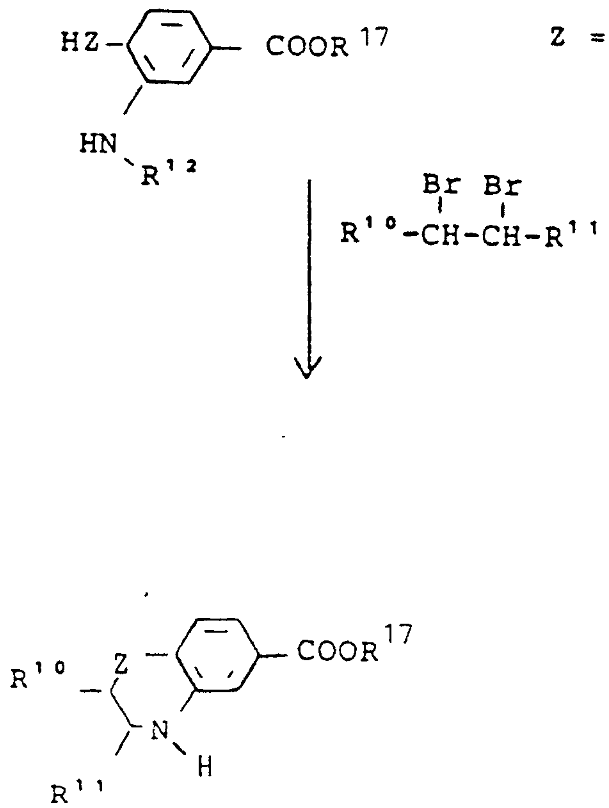

Z = O, NH, S

Literatur: H. Bartsch et al.. J. Heterocyclic Chem. 20 . 45 (1983).

4) Phenoxazinyl- und Phenothiazinylglycine

Ausgangsmaterial für die Synthese der Phenoxazin- und Phenothiazinglycinderivate ist Phenoxazin bzw. Phenothiazin.

Schema 5

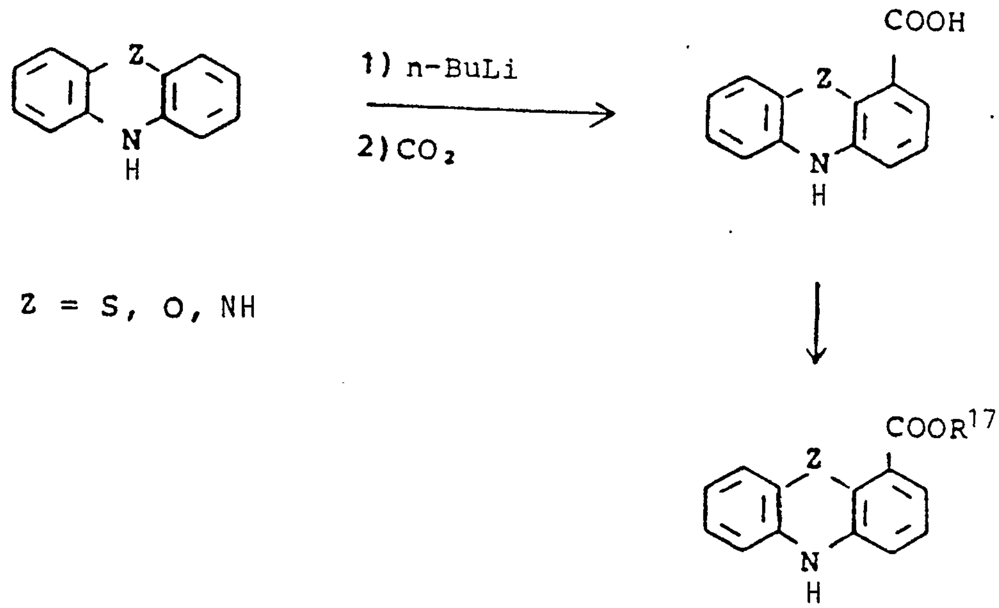


Literatur: M. Ionesen et al.. Advances in Heterocyclic Chemistry. Vol. 8. 83. Academic Press (1967).

5) Dibenzopyrrolyl- und Dibenzofurylglycine

Dibenzofuran und Dibenzopyrrol sind die Ausgangsstoffe für die Synthese von neuen anellierten Phenylglycinen. Die Zwischenverbindungen werden beispielsweise nach folgendem Syntheseschema hergestellt:

Schema 6

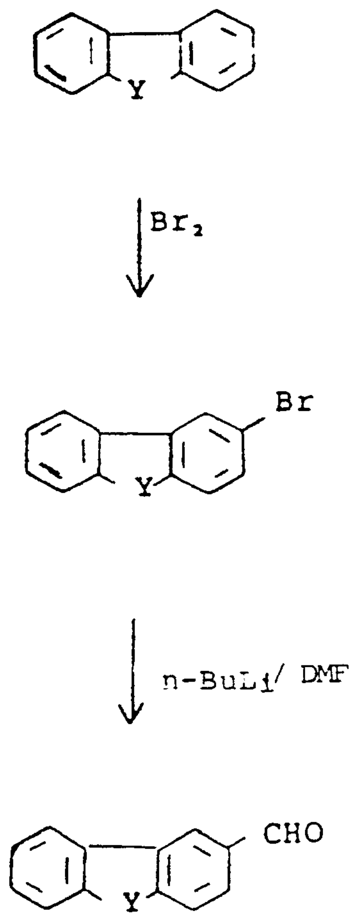


Literatur: M.L. Tedjamulia et al.. J. Heterocyclic Chem. 20 . 861 (1983)

6) Benzofuryl- und 1.2-Benzisoxazolyl-glycine

Ausgangsmaterial für die Synthese der Benzofuryl- und Benzisoxazolylglycine sind 3-Formyl-4-hydroxy-benzoesäureester. Die neuen anellierten Benzoesäurederivate werden beispielsweise nach folgendem Syntheseschema hergestellt.

Schema 7

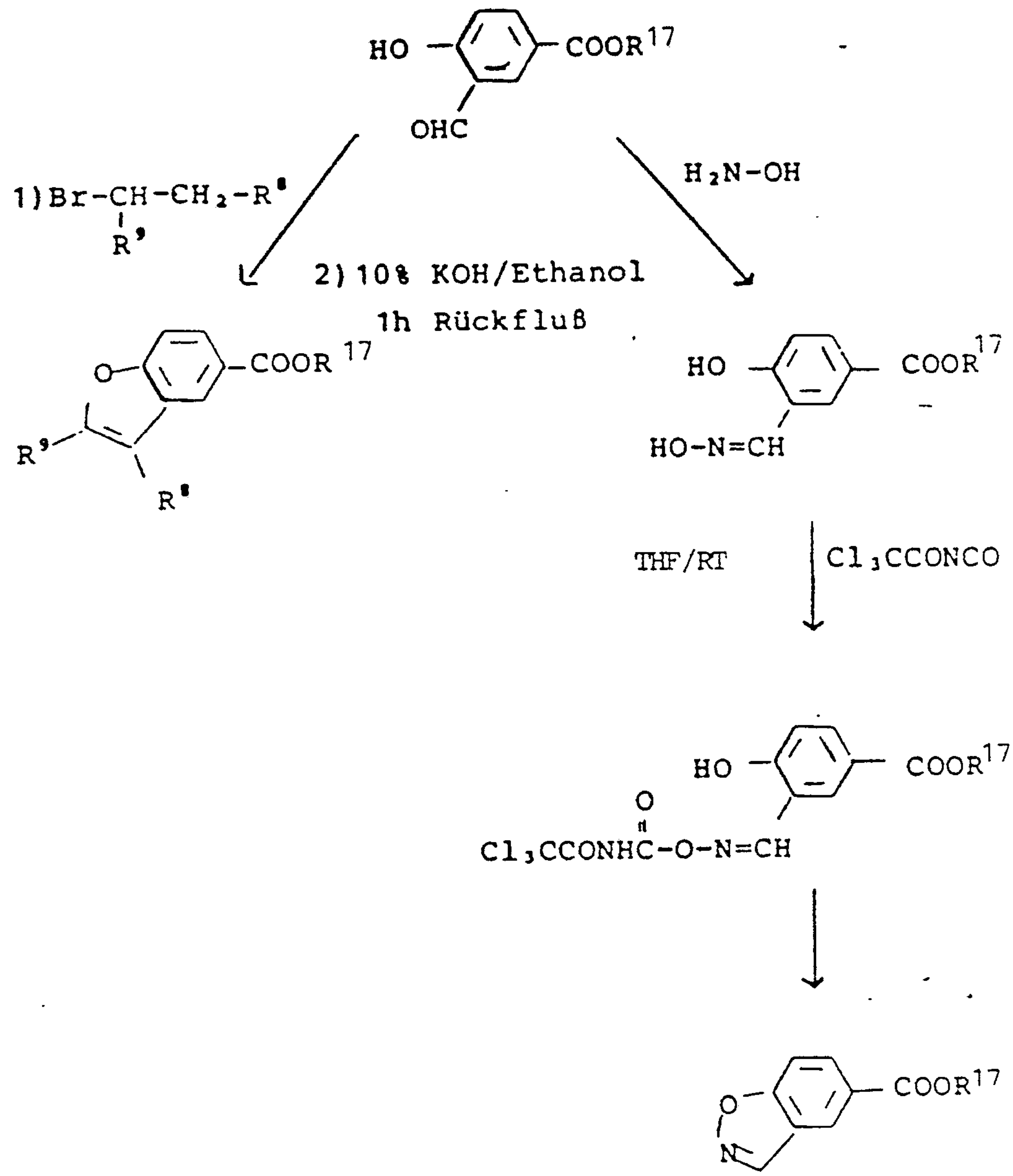


Literatur: R.C. Elderfield et al.. Benzofuran and its Derivatives, Heterocyclic Compounds. Vol. 2. 1 (1951). J. Wiley & Sons; P. Cagniant et al.. Advances in Heterocyclic Chemistry, Vol. 18. Academic Press (1975); G. Stokker, J. Org. Chem. 48 , 2613 (1983).

7) Indolyl-glycine

Für die Synthese von substituierten Indolylglycinen verwendet man 3-Methyl-4-nitrobenzoesäure. 2-Methyl-3-nitrobenzoesäure, 3-Nitro-4-Hydrazinobenzoesäure in Form ihrer Ester als Ausgangsmaterial. Die substituierten Indolcarbonsäureester werden beispielsweise nach der Leimgruber-Batcho- und Fischer-Indol-Synthese hergestellt:

Schema 8

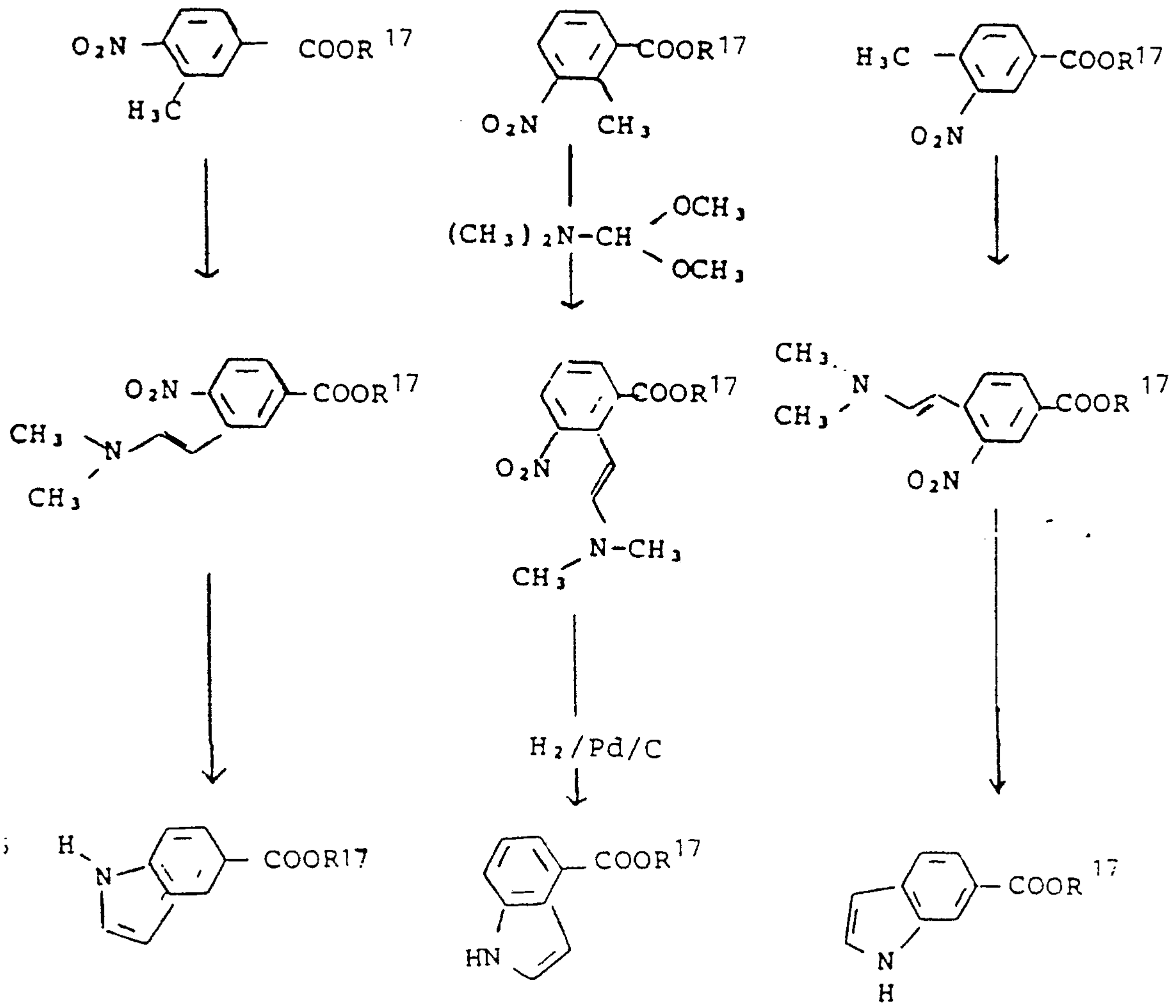

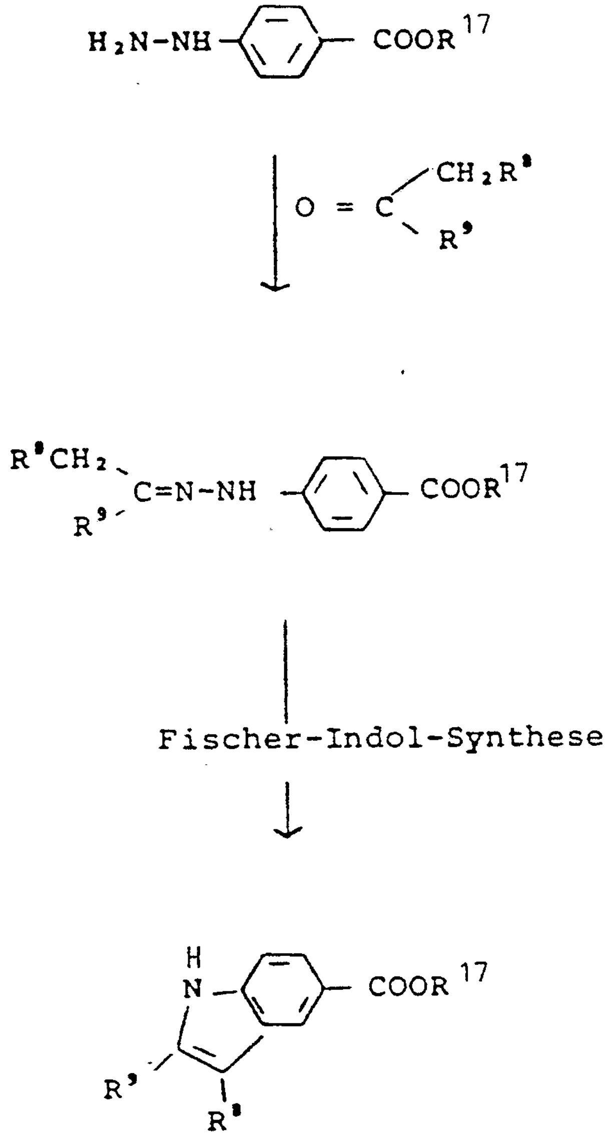


Literatur: R.K. Brown. Synthesis of the Indol Nucleus. Heterocyclic Compounds, Part I, 227, J. Wiley & Sons (1972); B. Robinson, Chemical Reviews 63 , 373 (1963); R. D. Clark et al., Heterocycles 22 . 195 (1984).

Die folgenden Cephalosporin-Grundkörper werden zur Herstellung der erfindungsgemäßen Verbindungen der Formel (I) eingesetzt:

1) Cephalosporin-Grundkörper (IIIa)

Die hier verwendeten typischen Cephalosporin-Grundkörper 7-Amino-3-methyl-3-cephem-4-carbonsäure (7-ADCA) und 7-Amino-3-chlor-3-cephem-4-carbonsäure (7-ACCA), die in J.MedChem. 12 . (310 C.W. Ryan et al.. 1969), US-Patent 3. 925. 372, Deutsche Offenlegungsschrift DE 2 606 196 und US-Patent 3. 994. 884 beschrieben sind, werden durch folgende Formel dargestellt:

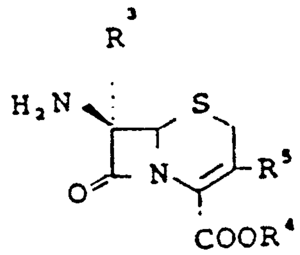

(IIIa)

| $R^3$ | $R^4$ | $R^5$ |
|---|---|---|
| H | H | $CH_3$ |
| H | t-Butyl | $CH_3$ |
| H | H | Cl |
| H | 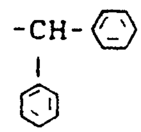 | Cl |
| H | H | H |
| $CH_3O$ | Trimethylsilyl | Cl |
| H | Allyl | $OCH_3$ |
| $CH_3S$ | t-Butyl | $CH_3$ |
| H | H (bzw. Benzhydryl) | $CH=CH_2$ |
| H | H | $CH=CH-CH_3$ |

Ganz besonders bevorzugte erfindungsgemäße Verbindungen der Formel I sind in den folgenden Tabellen angeführt.

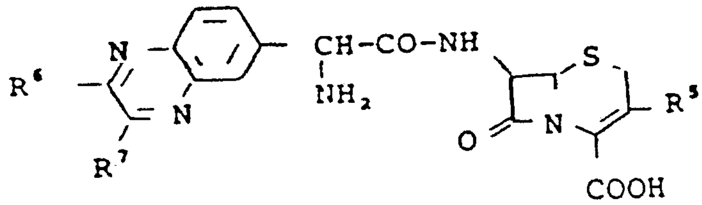

| $R^5$ | $R^6$ | $R^7$ |
|---|---|---|
| $CH_3$ | H | H |
| Cl | $NH_2$ | $NH_2$ |
| $OCH_3$ | HO | HO |
| $CH=CH_2$ | $NH_2$ | $NH_2$ |

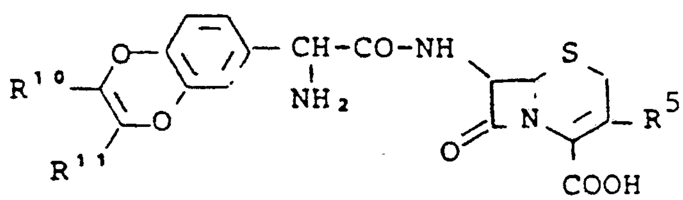

| $R^5$ | $R^{10}$ | $R^{11}$ |
|---|---|---|
| Cl | H | H |
| $OCH_3$ | H | H |
| Cl | $CH_3$ | $CH_3$ |

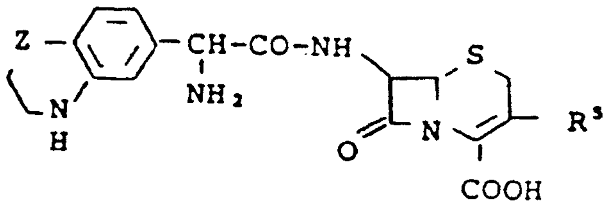

| Z | $R^5$ |
|---|---|
| O | Cl |
| O | $CH_3$ |
| NH | $OCH_3$ |
| NH | Cl |
| NH | $CH=CH_2$ |
| NH | $CH=CH-CH_3$ |

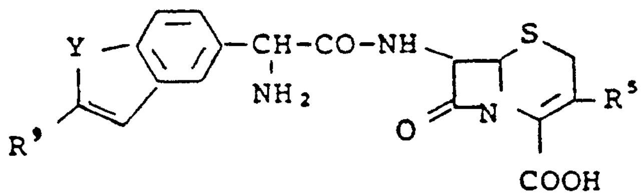

| Y | $R^5$ | $R^9$ |
|---|---|---|
| NH oder O | $CH_3$ | $CH_3$ |
| NH oder O | Cl | $CH_3$ |
| NH oder O | $CH=CH-CH_3$ | H |
| NH oder O | $OCH_3$ | 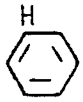 |
| NH oder O | Cl | 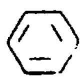 |
| NH oder O | $CH_3$ | 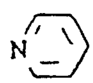 |
| NH oder O | Cl | 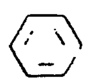 $-O-CH_2-$ |
| NH oder O | $OCH_3$ | 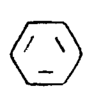 $-O-CH_2-$ |

Die erfindungsgemäßen Verbindungen der Formel I weisen bei geringer Toxizität ein breites antibakterielles Spektrum gegen gram-positive und gram-negative Keime auf, insbesondere gegen Staphylokokken, Streptokokken, Enterokokken und Haemophilus influenzae.

Bei parenteraler oder insbesondere oraler Verabreichung sind die neuen Verbindungen gegen Mikroorganismen, wie Staphylokokken,z.B. Staph. aureus, Staph. epidermidis, Streptokokken,wie z.B. Streptococcus pyogenes, Streptococcus faecalis, Enterobakteriaceen, z.B. Escherichia coli, Klebsiella, Salmonella, Shigella, Proteus, z.B. Proteus mirabilis, sehr gut wirksam.

Diese Eigenschaften ermöglichen ihre Verwendung als chemotherapeutische Wirkstoffe in der Human- und Tiermedizin. In der folgenden Tabelle sind die minimalen Hemmkonzentrationen (MHK-Werte. mg/ml) für das Beispiel 7 im Vergleich mit Cefaclor angegeben.

Die MHK-Werte werden durch den Agarverdünnungstest im festen Medium bestimmt wobei die Ablesung nach 18-24-stündiger Bebrütung bei 37°C erfolgt. Als Wuchsmedium. wird Isosensitest-Agar benutzt.

| Keime | Beispiel 7 | Cefaclor |
|---|---|---|
| Staph. 133 | 0.5 | 2 |
| Staph. 25022 | 0.5 | 2 |
| Staph. 25470 | 128 | >128 |
| Staph. E 25185 | 0.125 | 0.5 |
| Strept.faec. 27101 | 64 | >128 |
| Strept.faec. 113 | 32 | >128 |
| Enterococ.9790 | 16 | 64 |
| Enterococ. 27158 | 8 | 32 |

Beispielsweise können lokale und/oder systemische Erkrankungen behandelt und/oder verhindert werden, die durch die oben genannten Erreger oder durch deren Mischungen verursacht werden.

Als Krankheiten. die durch die erfindungsgemäßen Verbindungen verhindert, gebessert und/oder geheilt werden können, seien beispielsweise genannt:

Erkrankungen der Atmungswege und des Rachenraumes:

Otitis, Pharyngitis; Pneumonie; Peritonitis; Pyelonephritis; Cystitis; Endocarditis, Systeminfektionen, Bronchitis, Arthritis, lokale Infektionen.

Zur vorliegenden Erfindung gehören pharmazeutische Zubereitungen, die neben nichttoxischen, inerten pharmazeutisch geeigneten Trägerstoffen einen oder mehrere erfindungsgemäße Verbindungen enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet. daß die Zubereitungen in Form einzelner Teile. z.B. Tabletten. Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z.B. 1, 2, 3 oder 4 Einzeldosen oder 1/2. 1/3 oder 1/4 einer Einzeldosis enthalten. Eine Einzeldosis enthält vorzugsweise die Menge Wirkstoff. die bei einer Applikation verabreicht wird und die gewöhnlich einer ganzen. ein er halben. einem Drittel oder einem Viertel einer Tagesdosis entspricht.

Unter nichttoxischen. inerten pharmazeutisch geeigneten Trägerstoffen sind feste. halbfeste oder flüssige Verdünnungsmittel. Füllstoffe und Formulierungshilfsmittel jeder Art zu verstehen.

Als bevorzugte pharmazeutische Zubereitungen seien Tabletten. Dragees, Kapseln. Pillen. Granulate. Suppositorien. Lösungen. Suspensionen und Emulsionen, Pasten. Salben, Gele. Cremes. Lotions. Puder und Sprays genannt.

Tabletten. Dragees, Kapseln. Pillen und Granulate können den oder die Wirkstoffe neben den üblichen Trägerstoffen enthalten. wie (a) Füll- und Streckmittel. z.B. Stärken. Milchzucker. Rohrzucker. Glukose. Mannit und Kieselsäure (b) Bindemittel. z.B. Carboxymethylcellulose. Alginate. Gelatine. Polyvinylpyrrolidon. (c) Feuchthaltemittel. z.B. Glycerin. (d) Sprengmittel. z.B. Agar-Agar, Calciumcarbonat und Natriumcarbonat, (e) Lösungsverzögerer. z.B. Paraffin. (f) Resorptionsbeschleuniger. z.B. quarternäre Ammoniumverbindungen, (g) Netzmittel. z.B. Cetlalkohol, Glycerinmonostearat. (h) Adsorptionsmittel.z.B. Kaolin und Bentonit und

(i) Gleitmittel. z.B. Talkum. Calcium- und Magnesiumstearat und feste Polyethylenglykole oder Gemische der unter (a) bis (i) aufgeführten Stoffe.

Die Tabletten. Dragees. Kapseln. Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben. wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel z.B. Chlorfluorkohlenwasserstoffe enthalten.

Lösungen und Emulsionen können neben dem oder den Wirkstoffen die üblichen Trägerstoffe wie Lösungsmittel. Lösungsvermittler und Emulgatoren. z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel. z.B. Wasser, Ethylalkohol, Propylenglykol, Suspendiermittel, z.B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbessernde Zusätze, z.B. Pfefferminzöl oder Eukalyptusöl und Süßmittel, z.B. Saccharin, enthalten.

Die therapeutisch wirksamen Verbindungen sollen in den oben aufgegeführten pharmazeutischen Zubereitungen vorzugsweise in einer Konzentration von etwa 0.1 bis 99.5. vorzugsweise von etwa 0.5 bis 95 Gewichtsprozent der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die Wirkstoffe oder die pharmazeutischen Zubereitungen können lokal, oral, parenteral, intraperitoneal und/oder rectal, vorzugsweise oral oder parenteral wie intravenös oder intramuskulär appliziert werden.

Im allgemeinen hat es sich sowohl in der Human- als auch in der Veterinärmedizin als vorteihaft erwiesen. den oder die erfindungsgemäßen Wirkstoffe in Gesamtmengen von etwa 5 bis etwa 1000. vorzugsweise 10 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 250, insbesondere 3 bis 60 mg/kg Körpergewicht. Es kann jedoch auch erforderlich sein, von den genannten Dosierungen abzuweichen, und zwar in Abhängigkeit von der Art und dem Körpergewicht des zu behandelnden Objekts. der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in den üblichen Konzentrationen und Zubereitungen zusammen mit dem Futter bzw. mit Futterzubereitungen oder mit dem Trinkwasser gegeben werden. Dadurch kann eine Infektion durch gramnegative oder grampositive Bakterien

verhindert, gebessert und/oder geheilt werden und ebenso eine Förderung des Wachstums und eine Verbesserung der Verwertung des Futters erreicht werden.

Die neuen Verbindungen zeichnen sich durch starke antibakterielle Wirkungen, die in vivo und in vitro geprüft wurden, und durch orale Resorbierbarkeit aus.

Die erfindungsgemäßen Verbindungen können zum Zwecke der Erweiterung des Wirkungsspektrums und um eine Wirkungssteigerung speziell bei β-lactamasebildenden Bakterien zu erzielen mit anderen antimikrobiellen Wirkstoffen und Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit Aminoglykosidantibiotica. wie z.B. Gentamicin, Sisomicin, Kanamicin. Amikacin oder Tobramicin kombiniert werden.

Die Erfindung wird anhand der folgenden Beispiele weiter erläutert.

Beispiel 1

DL-7-(Chinolyl-6-glycylamido)-3-chlor-3-cephem-4-carbonsäure

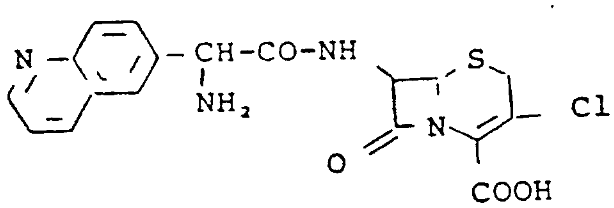

a) 6-Hydroxymethyl-chinolin (1a)

59,5 g (0.296 mol) Chinolincarbonsäure-6-ethylester werden in 970 ml Ether bei -70 °C mit 750 ml (0,9 mol) Diisobutylaluminium-hydrid (DIBAL, 20 %ig in Toluol, 1,2 molar) über Nacht gerührt. Anschließend läßt man die Temperatur auf -35 °C ansteigen und gibt 310 ml Kochsalzlösung hinzu, wobei die Temperatur langsam auf 20 °C kommt. Man rührt 3 Std. bei 20 °C, saugt Aluminiumhydroxid ab und wäscht mit Ether/Essigester nach. Die organische Phase wird mit 100 ml Kochsalzlösung gewaschen. über $Na_2SO_4$ getrocknet und eingedampft.

Rohausbeute: 40 g

Nach Chromatographie an Kieselgel (0,04-0,063 mm) mit dem Laufmittelsystem Petrolether/Essigester (3:1, 2 1), Petrolether/Essigester (1:1, 4 1) und Petrolether/Essigester (1:3, 7-8 1) erhält man reines Produkt.

Ausbeute: 33 g (70 %)

$C_{10}N_9NO$ (159,2)

NMR (DMSO): δ = 4,89 (s, 2H), 5,17 (s, 1H), 7,32 (dd, 1H), 7,62 (dd, 1H), 7,76 (s, 1H), 7,98-8,05 (m, 2H), 8,75 (dd, 1H) ppm.

b) Chinolin-6-aldehyd (1b)

32,6 g (0,205 mol) 1a werden in 1220 ml Methylenchlorid mit 122 g (1,4 mol) Mangan (IV)-oxid 3 Tage gerührt, vom Oxidationsmittel abgesaugt und das Filtrat bis zur Trcckne eingeengt.

Ausbeute: 26 g (81 %)

$C_{10}H_7NO$ (157,2)

NMR (DMSO): δ = 7,74 (dd, 1H), 8,2 (s,2H), 8,64 (dd, 1H), 8,68 (s, 1H), 9,12 (d, 1H), 10,25 (s, 1H) ppm.

c) 5-(Chinolyl-6 )-2.4-imidazolidindion (1c)

26 g (0,165 mol) 1b gelost in 165 ml Ethanol werden zu einer Losung von 12,2 g (0,248 mol) Natriumcyanid und 63.5 g (0,662 mol) Ammoniumcarbonat in 165 ml Wasser zugetropft und 22 Std. bei 60 °C gerührt. Nach Abdestillieren von Ethanol i.Vak. wird die Restlosung bei 0 °C mit 2N Salzsäure auf pH 2 angesauert, danach mit 2N Natronlauge auf pH 4.5 zurückgestellt und das ausgefallene Hydantoin abgesaugt.
Ausbeute: 27 g (76 %)
$C_{12}H_9N_2O_2$ (213.2)
NMR (DMSO): δ = 5,44 (s, 1H), 7,58 (dd, 1H), 7,75 (dd, 1H), 8,0 (s, 1H), 8,1 (d, 1H), 8,45 (dd, 1H), 8,6 (s, 1H), 8,95 (d,1H). 10,85 (breites s, 1H) ppm.

d) DL-α-Amino-α-chinolyl-6-essigsäure (1d)

27 g (0,127 mol) 1c und 144,2 g (0`457 mol) Bariumhydroxid in 1200 ml $H_2O$ werden 24 Std. bei 100 °C gerührt. Anschließend verdünnt man die Suspension mit 500 ml Wasser, leitet 2 Std. bei 100 °C $CO_2$ ein. saugt Bariumcarbonat ab und wäscht mit kochendem Wasser nach. Das Filtrat wird bis zur Trockne eingeengt.
Ausbeute: 16,6 g
Der Ruckstand wird in siedendem Wasser mit stark verdünnter Schwefelsäure versetzt, das ausgefallene Produkt abfiltriert und das Filtrat lyophylisiert.
Ausbeute: 12,1 g (38 %)
$C_{11}H_{10}N_2O_2$ 1/2 $H_2SO_4$ (251,2)
Ber. : C 52,58 H 4,41 N 11,4 S 6.38
Gef. : C 51,1 H 4,7 H 10,9 S 5,4
NMR (DCOOD): δ = 5,88 (s, 1H), 8,36 (t, 1H), 8,48 (d, 1H), 8,62 (d, 1H), 8,74 (s, 1H), 9,4-9,48 (m, 2H) ppm.

e) DL-α-t-Butyloxycarbonylamino-α-chinolyl-6-essigsäure (1e)

16,6 g (0,066 mol) 1d werden in 166 ml 2N Natronlauge, 166 ml $H_2O$ und 338 ml Dioxan gelöst, mit 43,2 g (0,198 mol) Di-t-butyl-dicarbonat innerhalb von 30 Minuten tropfenweise versetzt und 30 Std. bei Raumtemperatur gerührt. Dioxan wird abdestilliert, die Restlösung mit Essigester/Petrolether (1:1) gewaschen und die wäßrige Phase bei Eiskühlung mit 2N HC1 auf pH 2 angesäuert. Man extrahiert zweimal mit Essigester, wäscht die organische Phase mit Kochsalzlösung, trocknet über $Na_2SO_4$, und engt bis zur Trockne ein.
Ausbeute : 10,2 g (51 %)
$C_{16}H_{18}N_2O_4$ (302,3)

f) DL-7-(Chinolyl-6-glycylamido)-3-chlor-3-cephem-4-carbonsäure (1f)

Aktivierung der Percursorsäure:

2,4 g (7,93 mmol) 1e werden in 30 ml DMF gelöst, mit 1,11 ml (7,93 mmol) Triethylamin versetzt, bei -40 °C 0,977 ml (7, 93 mmol) Pivaloylchlorid tropfenweise zugespritzt und 3 Std. bei -30 °C bis -15 °C gerührt.

Zubereitung der Aminkomponente:

1,87 g (7,97 mmol) 7-Amino-3-chlor-3-cephem-4-carbonsäure (7-ACCA) werden in 15 ml THF und 7 ml $H_2O$ suspendiert und mit 10 %iger Triethylaminlösung in THF in Lösung gebracht (pH 8,3). Danach werden 2 ml DMF zugegeben, um eine einheitliche Phase zu bekommen.

Kupplung und Isolierung:

Die 7-ACCA-Lösung wird zum gebildeten Anhydrid bei -40° C zugespritzt und danach ohne Kältebad gerührt. Nach 1 Std. gibt man 5-10 ml $H_2O$ hinzu und stellt den pH mit 10 %iger Triethylamin in THF auf 7,3 ein. Nach weiteren 2 Std. gibt man ca. 140 ml $H_2O$ hinzu, versetzt unter Rühren mit Essigester und säuert bei 0° C auf pH 1,7 an. Die organische Schicht wird abgetrennt, mit Kochsalzlösung gewaschen, über $Na_2SO_4$ getrocknet und das Lösungsmittel i.Vak. abdestilliert.
Der Rückstand wird in wenig THF gelöst und in Petrolether eingerührt.
Ausbeute: 2,6 g (63 %)
$C_{23}H_{23}ClN_4O_6S$ (519,0)

2,5 g (4,82 mmol) Boc-geschütztes Cephalosporin werden in 25 ml Methylenchlorid gelöst, bei 0° C 0,5 ml Anisol und 25 ml Trifluoressigsäure (TFE) zugegeben und 15 Minuten ohne Kühlung gerührt. Anschließend wird die Lösung i.Vak. bis zur Trockene eingeengt und der Rückstand mit Ether versetzt. Das Trifluoracetat wird abgesaugt, mit Ether gewaschen und i.Vak. getrocknet. Zur Entfernung der Trifluoressigsäure wird die Substanz in 200 ml $H_2O$ gelöst und auf eine Säule mit Amberlite IRA-68 (Acetat-Form) gegeben. Man wäscht mit 150 ml Wasser nach und lyophylisiert das gesamte Eluat.
Ausbeute: 1,2 g (57 %)
$C_{18}H_{15}ClN_4O_4S$ $H_2O$ (436,9)
NMR (DCOOD): $\delta$ = 3,45-4,0 (mm, 2H), 5,28-5,38 (dd, 1H), 5,84-5,89 (dd, 1H), 5,98 (d, 1H), 8,35 (m, 1H), 8,46 (m, 1H), 8,58 (d, 1H), 8,76 (s, 1H), 9,38-9,48 (m, 2H) ppm.

Beispiel 2

DL-7-(Chinolyl-6-glycylamido)-3-methyl-3-cephem-4-carbonsäure

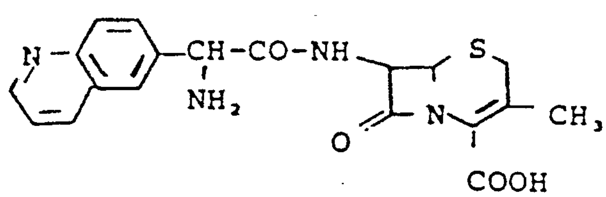

3,02 g (10 mmol) 1e, 1,53 g (10 mmol) 1-Hydroxybenztriazol werden unter Stickstoff in 15 ml THF gelöst 2,06 g (10 mmol) N,N'-Dicyclohexylcarbodiimid (DCC) gelöst in 10 ml THF werden bei 10° C zugegeben. Man rührt 2 Std. bei Raumtemperatur, gibt 2,7 g (10 mmol) 7-Amino-3-methyl-3-cephem-4-carbonsäure-t-butylester gelöst in 10 ml $CH_2Cl_2$ zur Lösung des aktivierten Esters bei 0° C hinzu und rührt danach ohne Kühlung über Nacht. Der ausgefallene Harnstoff wird abgesaugt, mit THF gevaschen und das Filtrat bis zur Trockene eingeengt. Der Rückstand wird in Essigester gelöst, mit $NaHCO_3$-Lösung und Wasser gewaschen. über $Na_2SO_4$ getrocknet und das Filtrat i.Vak. bis zur Trockne eingeengt.
Ausbeute: 1,9 g (34 %)
$C_{28}H_{34}N_4O_6S$ (554,7)

1,7 g (3,06 mmol) Boc-geschütztes Cephalosporin werden in 30 ml $CH_2Cl_2$ gelöst. bei 0° C 0,5, ml Anisol und 30 ml TFE zugegeben und 1 Std. bei Raumtemperatur gerührt. Anschließend wird das Lösungsmittel i.Vak. abdestilliert und der ölige Rückstand mit Ether verrieben. Das Trifluoracetat wird abgesaugt, mit Ether gewaschen, getrocknet, danach in 200 ml $H_2O$ aufgelöst und auf eine Säule mit Amberlite IRA-68 (Acetat-Form) gegeben. Man wäscht mit 100 ml Wasser nach und lyophylisiert das gesamte Eluat.
Ausbeute: 0,8 g (63 %)
$C_{19}H_{18}N_4O_4S$ $H_2O$ (416.4)
NMR (DCOOD): $\delta$ = 2,17 (d, 3H), 3,18-3,6 (m, 2H), 5,18-5,27 (dd, 1H), 5,86-6,02 (dd und s, 2H), 8,34 (m, 1H), 8,47 (d, 1H), 8,60 (t, 1H), 8,77 (s, 1H), 9.38-9,48 (m, 2H) ppm.

Beispiel 3

DL-7-(2,3-Dimethylchinoxalin-6-glycylamido)-3-chlor-3-cephem-4-carbonsäure

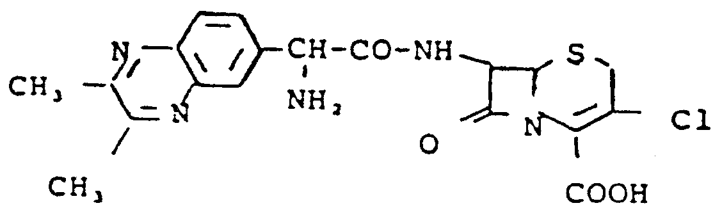

a) 2.3-Dimethylchinoxalin-6-carbonsäuremethylester (3a)

55.8 g (0.354 mol) 3.4-Diaminobenzoesäuremethylester und 30.5 g (0.354 mol) Diacetyl werden in 500 ml Toluol 3 Std. unter Rückfluß am Wasserabscheider erhitzt. Toluol wird i.Vak. abdestilliert. der Rückstand in Essigester unter Zusatz von A-Kohle in dar Wärme gelöst. die A-Kohle über einen Saitz-Filter abgetrennt und das Filtrat bis zur Trockne eingeengt.
Ausbeute: 72.6 g (95 %)
$C_{12}H_{12}N_2O_2$ (216.2)
NMR (DMSO): $\delta$ = 2.7 (s. 6H). 3,96 (s. 3H), 9,0 (d, 1H). 9,13 (d. 1H). 9.42 (s. 1H) ppm.

b) 2.3-Dimethyl-6-hydroxymethyl-chinoxalin (3b)

72,6 g (0,336 mol) 3a werden analog Beispiel 1a in 1100 ml THF mit 672 ml DIBAL (25 %ige Lösung in Toluol) bei -70 ° C zum Chinoxalinalkohol reduziert.
Ausbeute: 56,8 g (90 %)
$C_{11}H_{12}N_2O$ (188,2)
NMR (DMSO): $\delta$ = 2,6 (s, 6H), 4,67 (d, 2H), 5,45 (t, 1H), 7,62 (dd, 1H), 7,81-7,89 (s und d, 2H) ppm.

c) 2.3-Dimethylchinoxalin-6-carboxaldehyd (3c)

56,8 g (0,302 mol) 3b verden analog Beispiel 1b in 1000 ml $CH_2Cl_2$ mit 160 g (1,84 mol) Mangan-(IV)-oxid 4 Tage bei Raumtemperatur gerührt.
Rohausbeute: 54 g
Nach Chromatographie an Kieselgel mit dem Laufmittelsystem Toluol/Essigester (1 : 1) erhält man 36 g (64 %) reines Material.
$C_{11}H_{10}N_2O$ (186,2)
NMR (DMSO): $\delta$ = 2,75 (s, 6H), 8,13 (d, 1H), 8,69 (s, 1H), 10,27 (s, 1H) ppm.

d) 5-(2.3-Dimethylchinoxalin-6-yl)-4-imidazolidindion (3d)

36 g (0,193 mol) 3c werden analog Beispiel 1c in Methanol/Ethanol/Wasser mit 77`9 g (0,813 mol, 4,2 eq.) Ammoniumcarbonat und 14.7 g (0,230 mol, 1,55 eq) Natriumcyanid umgesetzt.
Ausbeute: 42,2 9 (85 %)
$C_{13}H_{12}N_4O_2$ (256,3)
NMR (DMSO): $\delta$ = 2,66 (s, 6H), 5,48 (s, 1H), 7,72 (dd, 1H), 7,9 (s, 1H), 8,02 (d, 1H), 8,66 (s, 1H), 10,98 (breites s, 1H) ppm.

e) DL-$\alpha$-t-Butyloxycarbonylamino-$\alpha$-(2.3-dimethylchinoxalin-6-yl)essigsäure (3e)

39,7 9 (0,155 mol) 3d werden mit 37,1 9 (1,55 mol) Lithiumhydroxid in 1000 ml Wasser 24 Stunden auf 100 ° C erhitzt. Die Lösung wird heiß filtriert, der Filterrückstand mit heißem Wasser nachgewaschen und

das Filtrat bei 0°C mit Salzsäure auf pH 2 angesäuert. Die Lösung wird mittels 2N Natronlauge auf pH 4,5 zurückgestellt und i.Vak. bis zur Trockene eingeengt. 2,5 g (10,8 mmol) 2.3-Dimethylchinoxalin-6-glycin werden in 10 %iger $NaHCO_3$-Lösung (50 ml) und 50 ml Dioxan mit 4,7 9 (21,6 mmol) Di-t-butyldicarbonat umgesetzt und analog Beispiel 1e aufgearbeitet.

Ausbeute: 1,8 g (50 %, THF/Petrolether)

$C_{17}H_{21}N_3O_4$ (331,4)

NMR (DMSO): $\delta$ = 1,42 (s, 9H), 2,7 (s, 6H), 5,42 (d,1H), 7,8 (d, 1H), 7,88 (d, 1H), 7,95-8,02 (s und d, 2H) ppm.

**f) DL-7-(2.3-Dimethylchinoxalin-6-glycylamido)-3-chlor-$\Delta^2/\Delta^3$-cephem-4-carbonsäure (3f)**

Zu einer auf -50°C gekühlten Lösung von 2,0 g (6,03 mmol) 3e in 15 ml DMF und 15 ml THF werden nacheinander 1,05 ml (6,03 mmol) Ethyldiisopropylamin und 0,467 ml (6,03 mmol) Methansulfonylchlorid langsam zugespritzt. Man rührt 45 Minuten bei -50°C und tropft eine Lösung (0°C) von 2,54 g (6,33 mmol) 7-Amino-3-chlor-3-cephem-4-carbonsäure-diphenylmethylester und 1,1 ml (6,33 mmol) Ethyldiisopropylamin in 12 ml THF und 12 ml $CH_2Cl_2$ zu. Es wird 15 Minuten bei -50°C und anschließend noch 45-60 Minuten ohne Kühlung nachgerührt. Danach wird das Lösungsmittel i.Vak. abdestilliert, der Rückstand in 300 ml Essigester gelöst, mit 0,1 N Salzsäure, Kochsalzlösung, $NaHCO_3$-Lösung und Wasser gewaschen. Nach dem Trocknen und Abdestillieren von Essigester erhält man 3,5 g (81 %) Rohprodukt, das in 100 ml Essigester unter Zusatz von Kieselgel gelöst und anschließend i.Vak. eingeengt wird. Das Pulver wird auf eine Säule (100 g Kieselgel, 0,04-0,063 mm) gegeben und nacheinander mit Toluol/Essigester (4:1) und Toluol/Essigester (3:1) eluiert.

Ausbeute: 1,7 g (40 %)

1,6 g Boc-geschütztes Cephalosporin werden analog Beispiel 2 deblockiert. Das Trifluoracetat wird mit Amberlite IRA-68 (Acetat-Form) ins Betain verwandelt.

Ausbeute: 0,45 g (42 %)

$C_{19}H_{18}ClN_5O_4S \bullet 2H_2O$ (483,9)

Die Trennung der 4 isomeren Cephalosporine erfolgt durch präparative HPLC (Hibar 250-25, RP-18, 7 µm) mit Laufmittel 0,1 % Trifluoressigsäure - Methanol (80:20).

D-Form (Peak III):

NMR (DCOOD): $\delta$ = 3,16 (d, 6H), 3,52 (d, 1H(), 3,86 (d, 1H), 5,31 (d, 1H), 5,97 (d, 1H), 6,02 (s, 1H), 8,4 (s, 1H) 8,56 (d, 1H), 8,71 (s, 1H) ppm.

Beispiel 4

DL-7-(Indolyl-4-glycylamido)-3-chlor-3-cephem-4-carbonsäure

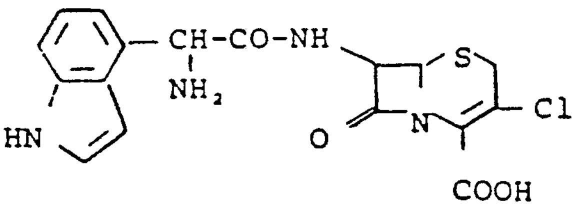

a) 5-(Indolyl-4-yl)-2.4-imidazolidindion (4a)

7,8 9 (53,7 mmol) Indol-4-carboxaldehyd werden analog Beispiel 1c in Ethanol und Wasser mit 4,05 g

(82,7 mmol) Natriumcyanid und 14,0 g (146 mmol) Ammoniumcarbonat umgesetzt.
Ausbeute: 6,4 9 (55 %)
$C_{11}H_9N_3O_2$ (215,2)
Ber.: C 61,4 H 4,2 N 19,5
Gef .: C 60,4 H 4,3 N 19,7

b) DL-α-Amino-α-indolyl-4-essigsäure (4b)

68,0 9 (0,316 mol) 4a werden analog Beispiel 1d mit 365,5 9 (1,152 mol) Bariumhydroxid in 2200 ml $H_2O$ 24 Std. bei 100 °C gerührt.
Ausbeute: 40 g (56 %)
$C_{10}H_{10}N_2O_2$ $2H_2O$ (226.2)
Bar.: C 53,10 H 6,24 N 12,39
Gef .: C 53,7 H 5,7 N 12,1
NMR (DMSO): δ = 4,6 (s, 1H), 6,64 (s, 1H), 7,02 (d, 2H), 7,32-7,38 (dd, 2H), 7,7-8,1 (breites s, 1H), 11,33 (s, 1H) ppm.

c) DL-α-t-Butyloxycarbonylamino-α-indolyl-4-essigsäure (4c)

6,0 g (26,5 mmol) 4b werden analog Beispiel 1e mit 11,5 g (53 mmol) Di-t-butyldicarbonat in Dioxan, Wasser und 2N Natronlauge umgesetzt.
Ausbeute: 5,2 g (68 %)
$C_{15}H_{18}N_2O_4$ (290.3)
NMR (DMSO): δ = 1,37 (s, 9H), 5,5 (d, 1H), 6,54 (s, 1H), 6,98-7,1 (m, 2H), 7,37 (m, 2H), 11,18 (s, 1H) ppm.

d) DL-7-[2-(t-Butyloxycarbonylamino)-indolyl-4-glyoylamido]-3-chlor-3-cephem-4-carbonsäure (4d)

7,05 g (24,3 mmol) 4c werden in 50 ml THF und 20 ml DMF mit 3,4 ml (24,3 mmol) Triethylamin, 2 Tropfen N-Methylmorpholin versetzt und bei -30 °C 3,15 ml (24,3 mmol) Chlorameisensäure-isobutylester zugegeben. Nach 60 Minuten bei -30 °C bis -10 °C gibt man eine vorgekühlte Triethylaminlösung von 6,55 9 (27,9 mmol) 7-ACCA in THF/ Wasser (3:1) hinzu. Man läßt 90 Minuten ohne Kühlung nachrühren und stellt den pH-Wert bei 7,2 ein. Man versetzt mit Essigester/ Wasser und säuert bei 0 °C auf pH 2 an. Die Essigesterphase wird abgetrennt, gewaschen über $Na_2SO_4$ getrocknet, bis auf 40 ml eingeengt und in Petrolether eingerührt. Das ausgefallene Produkt wird abgesaugt und getrocknet.
Ausbeute: 9,8 9 (80 %)
$C_{22}H_{23}ClN_4O_6S$ (506.9)
NMR (DMSO): δ = 1,4 (s, 9H) 3,6-4,04 (mm, 2H), 5,18-5,26 (dd, 2H), 5,72 (d, 1H), 5,81-5,88 (dd, 1H), 6,64 (s, 1H), 7,04 (m,2H), 7,38 (m, 2H), 9,22 (t, 1H), 11,18 (d, 1H) ppm.

e) DL -7-(Indolyl-4-glycylamido)-3-chlor-3-cephem-4-carbonsäure (4e)

10 g (19,7 mmol) 4d werden 15 Minuten ohne Zusatz von Anisol mit 50 ml TFE bei 10 °C gerührt. Das Trifluoracetat wird analog Beispiel If als Betain isoliert.
Ausbeute: 4,2 g (48 %)
$C_{17}H_{15}ClN_4O_4S$ 2 $H_2O$ (442.9)
NMR (DCOOD): δ = 3,43-3,95 (mm, 2H), 5,23-5,32 (dd, 1H), 5,85-5,94 (dd und d, 2H), 7,27 (m, 3H), 7,5 (s,1H), 7,68 (m, 1H) ppm.

Beispiel 5

D-7-( Indolyl-4-glycylamido)-3-chlor-3-cephem-4-carbonsäure (5a) und L-Form (5b)

Das Diastereomeren-Gemisch 4e wird an einer präparativen Merck-Säule (Hibar 250-25, RP-18, 7 µm, Laufmittel: 0,1 % Trifluoressigsäure-Methanol (85:15), 220 nm) in die D- und L-Form getrennt.

a) D-Form (Peak II):

NMR (DCOOD): δ = 3,5 (d, J = 18 Hz, 1H), 3,84 (d, J = 18 Hz, 1H, 5,25 (d, J = 5Hz, 1H), 5,89 (s, 1H), 5,93 (d, J = 5 Hz, 1H), 7,3 (d, 3H), 7,52 (s, 1H), 7,68 (t, 1H) ppm.

b) L-Form (Peak I):

NMR (DCOOD): δ = 3,72 (d J = 18 Hz, 1H), 3,95 (d, J = 18 Hz, 1H), 5,33 (d, J = 5 Hz, 1H), 5,88 (d, J = 5 Hz, 1H), 5,91 (s, 1H), 7,3 (m, 3H), 7,52 (s, 1H), 7,68 (m, 1H) ppm.

Beispiel 6

DL-7-( Indolyl-5-glycylamido)-3-chlor-3-cephem-4-carbonsäure

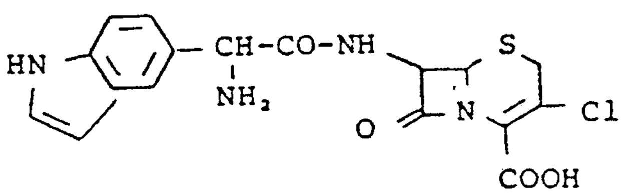

a) trans-3-[β-(Dimethylamino)vinyl]-4-nitrobenzoesäuremethylester (6a)

Eine Lösung von 86,4 g (0,443 mol) 3-Methyl-4-nitrobenzoesäuremethylester und 158,2 g (1,329 mol) N,N-Dimethylformamiddimethylacetal in 500 ml DMF wird 6 Std. bei 130 °C erhitzt. Danach wird DMF im Hochvakuum abdestilliert und der Rückstand i.Vak. bei 20 °C getrocknet.
Rohausbeute: 100,3 g (91 %)
$C_{12}H_{14}N_2O_4$ (250,3)
NMR (DMSO): δ = 2,96 (s, 6H), 3,96 (s, 3H), 5,8 (d J = 14 Hz, 1H), 7,1 (d, J = 14 Hz, 1H), 7,57 (dd, 1H), 7,85 (d, J = 8,5 Hz, 1H), 8,15 (s, 1H) ppm.

b) Indol-5-carbonsäuremethylester (6b)

227 g (1,106 mol) 6a (Rohprodukt) werden in 2000 ml Toluol 4 Std. mit 55 g Palladium auf Aktivkohle (10 % Pd) bei 10 bar in Gegenwart von Wasserstoff hydriert. Nach Abtrennen des Katalysators und Einengen der Toluol-Lösung wird das Indolderivat an Kieselgel (2300 g, 0,04-0,063 mm) mit dem Elutionsmittel Petrolether/Essigester (4:1) und Petrolether/Essigester (3: 1) chromatographiert.
Ausbeute: 58,9 g (30 %)
$C_{10}H_9NO_2$ (175.2)
NMR ($CDCl_3$): δ = 3,94 (s, 3H), 6,62 (s, 1H), 7,24 (t, 1H), 7,38 (d, 1H), 7,9 (d, 1H), 8,41 (s, 1H), 8,68 (breites s, 1H) ppm.

c) Indol-5-methanol (6c)

96 g (0,548 mol) 6b werden analog Beispiel 1a in 1800 ml Ether und 1391 ml (1,67 mol) DIBAL (1,2 molar in Toluol) behandelt. Nach Chromatographie an Kieselgel mit Gradienten-Elution von Petrolether/Essigester (3: 1) und Petrolether/Essigester (1:1) erhält man 39,3 g (49 %) reines Produkt.
$C_9H_9NO$ (147,2)

NMR ($CDCl_3$): $\delta$ = 3,91 (breites s, 1H), 4,72 (s, 2H). 6,45 (m, 1H), 7,17 (m, 2H), 7,37 (d, 1H), 7,6 (s, 1H), 9,88 (breites s, 1H) ppm.

d) Indol-5-carboxaldehyd (6d)

51,1 g (0,347 mol) 6c werden analog Beispiel 1b in 1900 ml $CH_2Cl_2$ und 200 ml THF mit 190 g (2,18 mol) Mangan-(IV)-oxid über Nacht bei 20 °C gerührt.
Ausbeute: 44,3 g (85 %)
$C_9H_7NO$ (145,2)
NMR ($CDCl_3$): $\delta$ = 6,71 (dd, 1H), 7,34 (dd, 1H), 7,5 (d, 1H), 7,79 (dd, 1H), 8,2 )d, 1H), 8,93 (breites s, 1H), 10,04 (s, 1H) ppm.

e) 5-(Indol-5-yl)-2,4-imidazolidindion (6e)

14,5 g (0,1 mol) 6d werden analog Beispiel 1c in Ethanol und Wasser mit 7,35 g (0,15 mol) Natriumcyanid und 38,4 9 (0,4 mol) Ammoniumcarbonat 2 Tage bei 60 °C gerührt.
Ausbeute: 17,3 g (81 %)
$C_{11}H_9N_3O_2$ (215,2)

f) DL-α-Amino-α-indolyl-5-essigsäure (6f)

17,3 g (0,08 mol) 6e werden analog Beispiel 1d mit 92,12 g (0,292 mol) Bariumhydroxid in 560 ml Wasser 24 Std. bei 100 °C gerührt.
Ausbeute: 12,1 g (67 %)
$C_{10}H_{10}N_2O_2$ 2 $H_2O$ (226.2)
Ber. : C 53,10 H 6.24 N 12,39
Gef. : C 54.8 H 5,0 H 12,4
NMR (NaOD): $\delta$ = 4,43 (s, 1H), 6,57 (d, 1H), 7,22 (d. 1H), 7,39 (d, 1H), 7,5 (d, 1H), 7,65 (s, 1H) ppm.

g) DL-α-t-Butyloxycarbonylamino-α-indolyl-5-essigsäure (6g)

14,0 g (0,0736 mol) 6f werden analog Beispiel 1e mit 64,2 g (0,294 mol) Di-t-butyldicarbonat in Dioxan, Wasser und 2N Natronlauge 30 Std. bei Raumtemperatur gerührt.
Ausbeute: 13,1 g (73 %)
$C_{15}H_{18}N_2O_4$ (290,3)
NMR (DMSO): $\delta$ = 1,38 (s,9H), 5,1 (d, 1H), 6,41 (s, 1H), 7,1 (dd, 1H), 7,34 (dd, 1H), 7,4 (d, 1H), 7,55 (s, 1H), 11,1 (s, 1H), 12,52 (breites s, 1H) ppm.

h) DL-7-Indolyl-5-glycylamido)-3-chlor-3-cephem-4-carbonsäure (6h)

3,5 9 (12,1 mmol) 6 g werden analog Beispiel 4d in 20 ml THF und 20 ml DMF mit 1,69 ml (12,1 mmol) Triethylamin. 2 Tropfen N-Methylmorpholin, 1,57 ml (12,1 mmol) Chlorameisensäure-isobutylester und 2,84 g (12,1 mmol) 7-ACCA umgesetzt.
Ausbeute: 4,6 g (75 %)
$C_{22}H_{23}ClN_4O_6S$ (506,9)
4 g (7,89 mol) Boc-geschütztes Cephalosporin werden in 40 ml $CH_2Cl_2$, 40 ml TFE und 10 ml Thioanisol 10 Minuten bei 0 °C unter Argon gerührt. Die Entfernung der Trifluoressigsaure und die Bildung des Betains erfolgt analog Beispiel 1g.
Ausbeute: 1 g (29 %)
$C_{17}H_{15}ClN_4O_4S$ 2 $H_2O$ (442.9)
NMR (DCOOD): $\delta$ = 3,5-4,02 (mm, 2H), 5,28-5,4 (dd, 1H), 5,65-5,92 (mm, 2H), 7,27 (m, 2H), 7,37 (m, 2H), 7,68 (m, 1H) ppm.

Beispiel 7

DL-7-(Benzofuryl-5-glycylamido)-3-chlor-3-cephem-4-carbonsäure

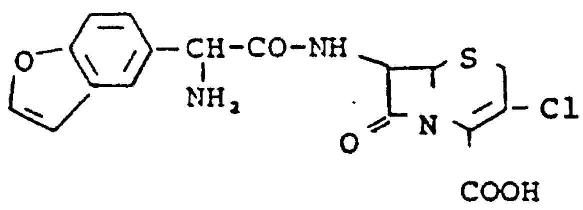

a) 5-Hydroxymethyl-benzofuran (7a)

5,8 g (32,9 mmol) Benzofuran-5-carbonsäuremethylester werden analog Beispiel 1 g in 50 ml THF mit 64,5 ml (98,6 mmol) DIBAL (1,53 molar) bei -70° C über Nacht gerührt.
Ausbeute: 3,9 g (80 %)
$C_9H_8O_2$ (148,2)
NMR (DMSO): δ = 4,6 (d, 2 H), 5,24 (t, 1H), 6,96 (d, 1H), 7,3 (dd, 1H), 7,58 (d, 1H), 7,62 (s, 1H), 7,99 (d, 1H), ppm.

b) Benzofuran-5-carboxaldehyd (7b)

5,3 g (35,8 mmol) 7a werden analog Beispiel 1b in 360 ml Methylenchlorid mit 19,7 g (227 mmol) Mangan-(IV)-oxid 2 Tage bei Raumtemperatur gerührt.
Ausbeute: 4,6 g (88 %)
$C_9H_6O_2$ (146,1)
NMR (DMSO): δ = 7,21 (d, 1H), 7,84 (d, 1H), 7,96 (dd, 1H), 8,22 (d, 1H), 8,32 (s, 1H), 10,12 (s, 1H) ppm.

c) DL-α-Amino-α-(benzofur-5-yl)-essigsäure (7c)

19,0 g (0,13 mol) 7b werden analog Beispiel 1c in Ethanol und Wasser mit 9,6 g (0,195 mol) Natriumcyanid und 49,9 g (0,52 mol) Ammoniumcarbonat 18 Std. bei 60° C gerührt.
Ausbeute: 21,3 g (76 %)
$C_{11}H_8N_2O_3$ (216.2)
Das Hydantoin wird mit 10 %iger Natronlauge 30 Std. bei 100° C behandelt, anschließend mit halbkonz. Salzsäure bei Eiskühlung auf pH 2 angesäuert und danach mittels 2N Natronlauge auf pH 4,5 zurückgestellt.
Ausbeute: 11,3 g (60 %)
$C_{10}H_9NO_3$ (191,2)
NMR (DCOOD): δ = 5,66 (s, 1H), 7,0 (d, 1H), 7,58 (dd. 1H), 7,76 (d, 1H), 7,91 (d, 1H), 7,96 (d, 1H) ppm.

d) DL-α-(t-Butyloxycarbonylamino-α-benzofur-5-yl)essigsäure (7d)

6,0 g (0.0314 mol) 7c werden analog Beispiel le mit 27,4 g (0.126 mol) Di-t-butyldicarbonat in 60 ml Wasser, 60 ml 2N Natronlauge und 120 ml Dioxan über Nacht gerührt.
Ausbeute: 6.6 g (73 %)
$C_{15}H_{17}NO_5$ (291.3)
NMR (DMSO): δ = 1,38 (s, 9H), 5,22 (d, 1H), 7,0 (d, 1H), 7,38 (dd, 1H), 7,59 (d, 1H), 7,71 (s, 1H), 8,04 (d, 2H) ppm.

e) DL-7-Benzofuryl-5-glycylamido)-3-chlor-3-cephem-4-carbonsäure (7e)

2,0 g (6,86 mmol) 7d werden analog Beispiel 4d in THF und DMF mit 0,96 ml (6,86 mmol) Triethylamin, 3 Tropfen H-Methylmorpholin, 0,89 ml (6,86 mmol) Chlorameisensäureisobutylester und 1,77 g (7,55 mmol) 7-ACCA umgesetzt.
Ausbeute: 1,6 g (46 %)
$C_{22}H_{22}ClN_3O_7S$ (507 ,9)
1,6 g (3,15 mmol) Boc-geschütztes Cephalosporin werden analog Beispiel 1g mit TFE ( + 3 Tropfen Anisol) deblockiert und mit Amberlite IRA-68 (Acetat-Form) von TFE befreit.
Ausbeute: 0,9 g (65 %)
$C_{17}H_{14}ClN_3O_5S$ 2 $H_2O$ (443.9)
NMR (DCOOD): δ = 3,55-4,06 (mm, 2H), 5,33-5,4 (dd, 1H). 5,75 (s, 1H), 5,9-6,0 (dd, 1H), 7,01 (s, 1H), 7,61 (m, 1H), 7,74-7,8 (m, 1H), 7,92-7,98 (m, 2H) ppm.

## Beispiel 8

D-7-(Benzofuryl-5-glycylamido)-3-chlor-3-cephemd-4-carbonsäure (8a) und L-Form (8b)

Präparative HPLC-Trennung von 7e:

Säule: Zorbax DuPont 250-21,2 (ODS, 220 nm)
Laufmittel: 800 ml $H_2O$ - 200 ml Acetonitril - 1 ml TFE
Aufgabe: 0,7 g, jeweils 30-50 mg in 2 ml Probeschleife
Fluß: 12,5 ml/min.

b) L-Form (Peak I):

Ausbeute: 186 mg
$C_{17}H_{14}ClN_3O_5S$ $CF_3COOH$ $H_2O$ (539.9)
Ber.: C 42,27 H 3,17 S 5,94 F 10,55
Gef .: C 42,8 H 3,2 S 6,2 F 9,5
NMR (DCOOD): δ = 3,73 (d, J=18 Hz, 1H), 3,96 (d, J=18 Hz, 1H), 5,32 (d, J=5 Hz, 1H), 5,68 (s, 1H), 5,84 (d, J=5 Hz, 1H), 6,92 (s, 1H), 7,52 (d, J=8,0 Hz, 1H), 7,68 (d, J=8,0 Hz, 1H), 7,84 (s, 1H), 7,9 (s, 1H) ppm.

a) D-Form (Peak II):

Ausbeute: 165 mg
NMR (DCOOD): δ = 3,5 (d, J=18 Hz, 1H), 3,84 (d, J=18 Hz. 1H), 5,25 (d, J=5 Hz, 1H), 5,65 (s, 1H) 5,9 (d, J=5 Hz, 1H), 6,92 (s, 1H), 7,5 (d, J=8 Hz, 1H), 7,68 (d, J=8 Hz, 1H), 7,84 (s, 1H), 7,99 (s, 1H) ppm.

## Beispiel 9

DL-7-(Benzofuryl-5-glycylamido)-3-methyl-3-cephem-4-carbonsäure

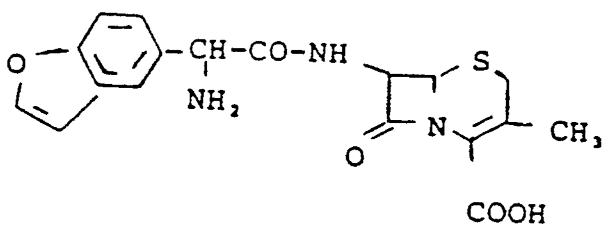

1 g (3,43 mmol) 7d werden analog Beispiel 3f in 16 ml $CH_2Cl_2$ mit 0,6 ml (3,43 mmol) Ethyldiisopropylamin, 0,265 ml (3,43 mmol) Methansulfonylchlorid und 0,927 g (3,43 mmol) 7-Amino-3-methyl-3-cephem-4-carbonsäure-t-butylester, der in 14 ml $CH_2Cl_2$ in Gegenwart von 0.597 ml (3,43 mmol) Ethyldiisopropylamin gelöst wird. umgesetzt.
Ausbeute: 1,1 g (59 %)
$C_{27}H_{33}N_3O_7S$ (543.6)
1,1 g (2 mmol) Boc-geschütztes Cephalosporin werden analog Beispiel 3f deblockiert und an einer Amberlite IRA-68-Säule (Acetat-Form) von TFE befreit.
Ausbeute: 350 mg (42 %)
$C_{18}H_{17}N_3O_5S$ 2 $H_2O$ (411.4)
NMR (/DCOOD): $\delta$ = 2,2 (d, 3H), 3,22-3,64 (mm, 2H), 5,18-5,24 (dd, 1H), 5,70 (d, 1H), 5,78-5,88 (dd, 1H) 6,97 )m, 1H), 7,56 (d, 1H), 7,69-7,76 (m, 1H), 7,88 (m, 1H), 7,94 (s, 1H) ppm.

## Ansprüche

**1.** $\beta$-Lactamverbindungen der allgemeinen Formel (I)

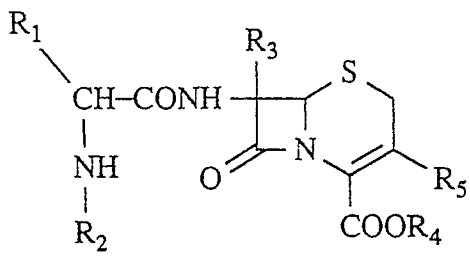

(I)

worin
$R_5$ für Wasserstoff, Halogen, Azido oder für geradkettiges, verzweigtes oder cyclisches, gesättigtes oder ungesättigtes Alkyl mit bis zu 6 C-Atomen steht, das gegebenenfalls substituiert ist durch Halogen, $OCONH_2$, $C_1$-$C_5$-Alkoxy, $C_2$-$C_7$-Acyloxy oder $C_1$-$C_5$-Alkylthio steht,
$R_1$ für einen Rest der Formel

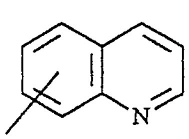 , 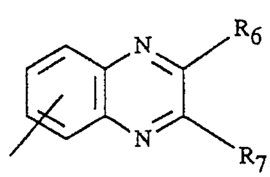 , 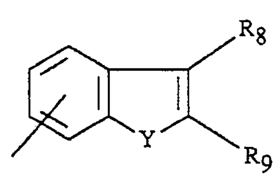

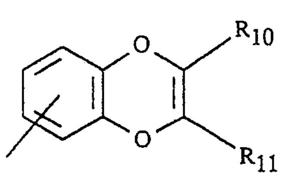 , 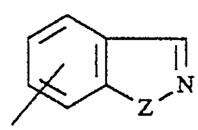 , 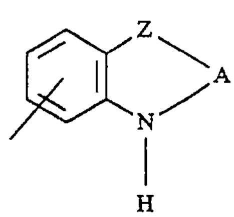 steht,

worin
$R_6$, $R_7$ gleich oder verschieden sind und für Wasserstoff, für $C_6$-$C_{10}$-Aryl, für eine Aminogruppe oder für Hydroxy stehen,
$R_8$, $R_9$ gleich oder verschieden sind und für Wasserstoff, für Hydroxy, für eine Aminogruppe, für

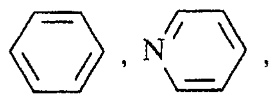

für Alkyl mit bis zu 8 C-Atomen, das substituiert ist durch Phenyloxy, für Phenyl, das substituiert ist durch Chlor oder Amino, oder für Alkyl mit bis zu 12 C-Atomen stehen oder
$R_8$ und $R_9$ zusammen für die Gruppe

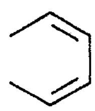

stehen,
A für die Gruppe

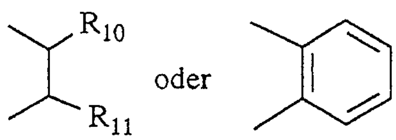

steht,
$R_{10}$, $R_{11}$ gleich oder verschieden sind und für Wasserstoff, für Alkyl mit bis zu 12 C-Atomen,
Y für Sauerstoff oder -NH steht,
Z für Sauerstoff, Schwefel oder -NH steht,
und worin
$R_2$ für Wasserstoff oder eine der folgenden Aminoschutzgruppen, tert.Butoxycarbonyl (Boc), Carbobenzoxy (Cbz), 2-Methoxycarbonyl-1-methylvinyl, Trityl (Trt), Benzyl (Bzl), Benzyloxycarbonyl (Z), Formyl oder Chloracetyl,
$R_3$ für Wasserstoff, für Alkoxy, Alkylthio mit jeweils bis zu 5 C-Atomen, und
$R_4$ für Wasserstoff oder für eine der folgenden Carboxyschutzgruppen tert.-Butyl, Decyl, 2,2,2-Trichlorethyl, Benzyl, 4-Methoxybenzyl, 4-Nitrobenzyl, Triphenylmethyl, Diphenylmethyl, Acetoxymethyl, Pivaloyloxymethyl, Allyl oder Trimethylsilyl für Alkaliionen oder Ammoniumionen oder für einen Rest der Formel $-CH_2-O-CO-C(CH_3)_3$ oder $-CH(CH_3)-O-CO-O-C_2H_5$ steht.

2. β-Lactamverbindungen nach Anspruch 1, dadurch gekennzeichnet, daß $R_5$ Fluor, Chlor, Brom, Methoxy, Methylthio, Trifluormethyl, Methoxymethyl oder für gegebenenfalls durch Halogen oder $OCONH_2$ substituiertes Methyl, Vinyl, $-CH=CH-CH_3$, $-CH=CH-C_2H_5$ steht.

3. Verfahren zur Herstellung von β-Lactamverbindungen nach Anspruch 1, dadurch gekennzeichnet; daß man Verbindungen der allgemeinen Formel IIa

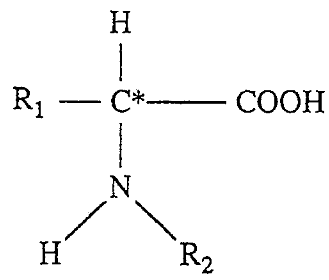

(IIa)

in welcher

$R_1$ die in Anspruch 1 angegebene Bedeutung hat und in welcher

$R_2$ für eine in der $\beta$-Lactam-Chemie übliche Aminoschutzgruppe steht,

nach Aktivierung der Carboxylgruppe durch Überführung in ein gemischtes Arhydrid, nach Überführung in das Mesylat oder nach Überführung in einen aktivierten Ester mit Verbindungen der allgemeinen Formel (III)

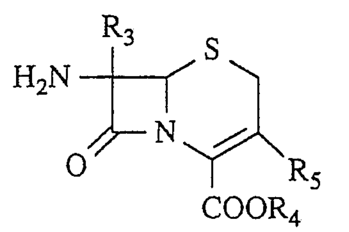

(III)

in welcher

$R_3$ und $R_4$ die in Anspruch 1 angegebene Bedeutung haben, zur Umsetzung bringt, dann gegebenenfalls Schutzgruppen abspaltet und die gewünschten Salze oder aus den Salzen die freien Säuren herstellt.

**4.** $\beta$-Lactamverbindungen der allgemeinen Formel (I) nach Anspruch 1 zur Verwendung bei der Bekämpfung von Krankheiten des menschlichen und tierischen Körpers.

**5.** Arzneimittel enthaltend $\beta$-Lactamverbindungen der allgemeinen Formel (I) nach Anspruch 1.

**6.** Verwendung von $\beta$-Lactamverbindungen der allgemeinen Formel (I) nach Anspruch 1 zur Herstellung von Arzneimitteln.

Patentansprüche für folgenden Vertragsstaat : AT

**1.** Verfahren zur Herstellung von $\beta$-Lactamverbindungen der allgemeinen Formel (I)

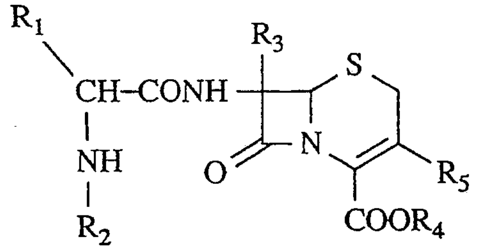

(I)

worin

$R_5$ für Wasserstoff, Halogen, Azido oder für geradkettiges, verzweigtes oder cyclisches, gesättigtes oder ungesättigtes Alkyl mit bis zu 6 C-Atomen steht, das gegebenenfalls substituiert ist durch Halogen, $OCONH_2$, $C_1$-$C_5$-Alkoxy, $C_2$-$C_7$-Acyloxy oder $C_1$-$C_5$-Alkylthio steht,

$R_1$ für einen Rest der Formel

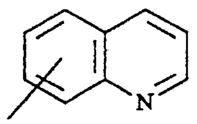 , 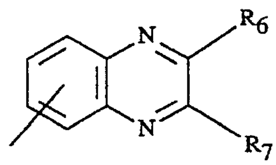 , 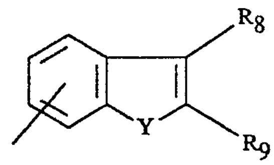

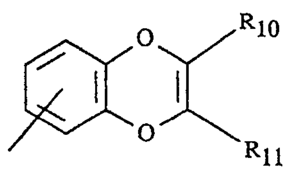 , 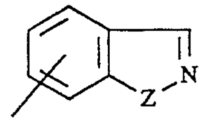 , 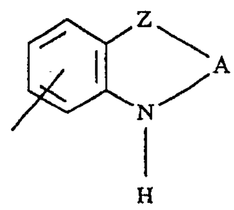

steht, worin
$R_6$, $R_7$ gleich oder verschieden sind und für Wasserstoff, für $C_6$-$C_{10}$-Aryl, für eine Aminogruppe oder für Hydroxy stehen,
$R_8$, $R_9$ gleich oder verschieden sind und für Wasserstoff, für Hydroxy, für eine Aminogruppe, für

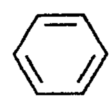 , 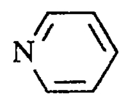 ,

für Alkyl mit bis zu 8 C-Atomen, das substituiert ist durch Phenyloxy, für Phenyl, das substituiert ist durch Chlor oder Amino, oder für Alkyl mit bis zu 12 C-Atomen stehen oder
$R_8$ und $R_9$ zusammen für die Gruppe

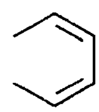

stehen,
A für die Gruppe

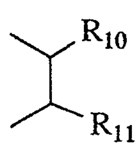 oder 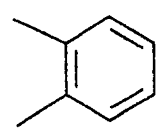

steht,
$R_{10}$, $R_{11}$ gleich oder verschieden sind und für Wasserstoff, für Alkyl mit bis zu 12 C-Atomen,
Y für Sauerstoff oder -NH steht,
Z für Sauerstoff, Schwefel oder -NH steht,
und worin
$R_2$ für Wasserstoff oder eine der folgenden Aminoschutzgruppen,
tert.-Butoxycarbonyl (Boc), Carbobenzoxy (Cbz), 2-Methoxycarbonyl-1-methylvinyl, Trityl (Trt), Benzyl (Bzl), Benzyloxycarbonyl (Z), Formyl oder Chloracetyl,
$R_3$ für Wasserstoff, für Alkoxy, Alkylthio mit jeweils bis zu 5 C-Atomen, und
$R_4$ für Wasserstoff oder für eine der folgenden Carboxyschutzgruppen
tert.-Butyl, Decyl, 2,2,2-Trichlorethyl, Benzyl, 4-Methoxybenzyl, 4-Nitrobenzyl, Triphenylmethyl, Diphenylmethyl, Acetoxymethyl, Pivaloyloxymethyl, Allyl oder Trimethylsilyl für Alkaliionen oder Ammoniumionen oder für einen Rest der Formel $-CH_2-O-CO-C(CH_3)_3$ oder $-CH(CH_3)-O-CO-O-C_2H_5$ steht,
**dadurch gekennzeichnet,** daß man Verbindungen der allgemeinen Formel (IIa)

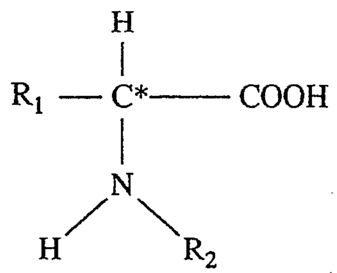

(IIa)

in welcher

$R_1$ die oben angegebene Bedeutung hat und in welcher

$R_2$ für eine in der β-Lactam-Chernie übliche Aminoschutzgruppe steht, nach Aktivierung der Carboxylgruppe durch Überführung in ein gemischtes Anhydrid, nach Überführung in das Mesylat oder nach Überführung in einen aktivierten Ester mit Verbindungen der allgemeinen Formel (III)

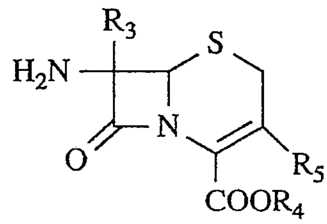

(III)

in welcher

$R_3$ und $R_4$ die oben angegebene Bedeutung haben, zur Umsetzung bringt, dann gegebenenfalls Schutzgruppen abspaltet und die gewünschten Salze oder aus den Salzen die freien Säuren herstellt.

2. Verfahren zur Herstellung von β-Lactamverbindungen nach Anspruch 1, **dadurch gekennzeichnet,** daß

   $R_5$ Fluor, Chlor, Brom, Methoxy, Methylthio, Trifluormethyl, Methoxymethyl oder für gegebenenfalls durch Halogen oder $OCONH_2$ substituiertes Methyl, Vinyl, $-CH=CH-CH_3$, $-CH=CH-C_2H_5$ steht.

3. β-Lactamverbindungen der allgemeinen Formel (I) nach Anspruch 1 zur Verwendung bei der Bekämpfung von Krankheiten des menschlichen und tierischen Körpers.

4. Arzneimittel enthaltend β-Lactamverbindungen der allgemeinen Formel (I) nach Anspruch 1.

5. Verwendung von β-Lactamverbindungen der allgemeinen Formel (I) nach Anspruch 1 zur Herstellung von Arzneimitteln.

## Claims

1. β-Lactam compounds of the general formula (I)

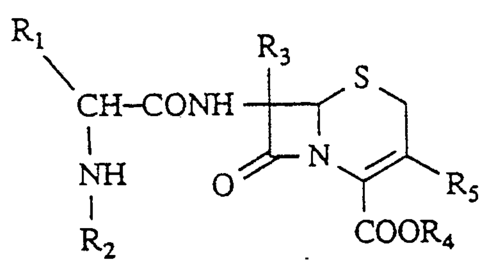

(I)

in which

$R_5$ represents hydrogen, halogen, azido or straight-chain, branched or cyclic, saturated or unsaturated alkyl having up to 6 C atoms, which is optionally substituted by halogen, $OCONH_2$, $C_1$-$C_5$-alkoxy, $C_2$-$C_7$-acyloxy or $C_1$-$C_5$-alkylthio,

$R_1$ represents a radical of the formula

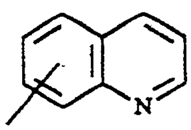 , 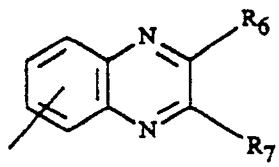 , 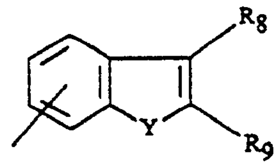

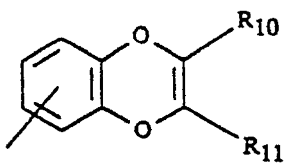 , 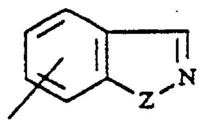 , 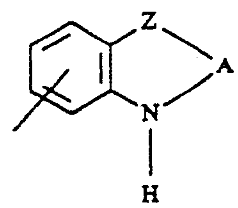

in which

$R_6$ and $R_7$ are identical or different and represent hydrogen, $C_6$-$C_{10}$-aryl, an amino group or hydroxyl,

$R_8$ and $R_9$ are identical or different and represent hydrogen, hydroxyl, an amino group,

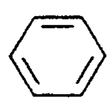 , 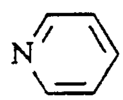 ,

alkyl having up to 8 C atoms, which is substituted by phenyloxy,
phenyl which is substituted by chlorine or amino, or
alkyl having up to 12 C atoms or
$R_8$ and $R_9$ together represent the group

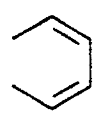

A represents the group

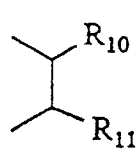 or 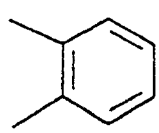

$R_{10}$ and $R_{11}$ are identical or different and represent hydrogen or alkyl having up to 12 C atoms,

Y represents oxygen or -NH,

S represents oxygen, sulphur or -NH,

and in which

$R_2$ represents hydrogen or one of the following amino protecting groups,
tert.-butoxycarbonyl (Boc), carbobenzoxy (Cbz), 2-methoxycarbonyl-1-methylvinyl, trityl (Trt), benzyl (Bzl), benzyloxycarbonyl (Z), formyl or chloroacetyl,

$R_3$ represents hydrogen, alkoxy or alkylthio in each case having up to 5 C atoms, and

$R_4$ represents hydrogen or one of the following carboxyl protecting groups tert.-butyl, decyl, 2,2,2-trichlorethyl, benzyl, 4-methoxybenzyl, 4-nitrobenzyl, triphenylmethyl, diphenylmethyl, acetoxymethyl, pivaloyloxymethyl, allyl or trimethylsilyl, or alkali metal ions or ammonium ions or a radical of the formula $-CH_2-O-CO-C(CH_3)_3$ $-CH(CH_3)-O-CO-O-C_2H_5$.

2. β-Lactam compounds according to Claim 1, characterized in that
$R_5$ represents fluorine, chlorine, bromine, methoxy, methylthio, trifluoromethyl, methoxymethyl or methyl which is optionally substituted by halogen or $OCONH_2$, vinyl, $-CH=CH-CH_3$ or $-CH=CH-C_2H_5$.

3. Process for the preparation of β-lactam compounds according to Claim 1, characterized in that compounds of the general formula IIa

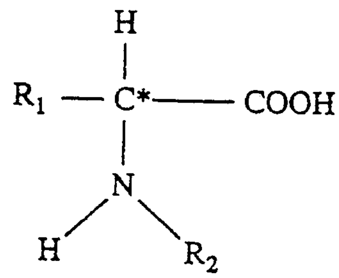

(IIa)

in which
$R_1$ has the meaning indicated in Claim 1 and in which
$R_2$ represents an amino protecting group customary in β-lactam chemistry,
are reacted, after activation of the carboxyl group by conversion into a mixed anhydride, after conversion into the mesylate or after conversion into an activated ester, with compounds of the general formula (III)

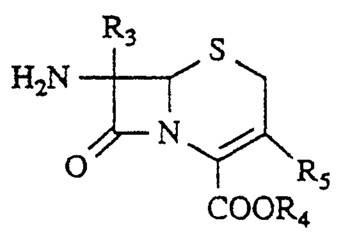

(III)

in which
$R_3$ and $R_4$ have the meaning indicated in Claim 1, then, if desired, protecting groups are removed and the desired salts are prepared, or, from the salts, the free acids.

4. β-Lactam compounds of the general formula (I) according to Claim 1 for use in the control of diseases of the human and animal body.

5. Medicaments containing β-lactam compounds of the general formula (I) according to Claim 1.

6. Use of β-lactam compounds of the general formula (I) according to Claim 1 for the production of medicaments.

Claims for the following Contracting State : AT

1. Process for the production of β-lactam compounds of the general formula (I)

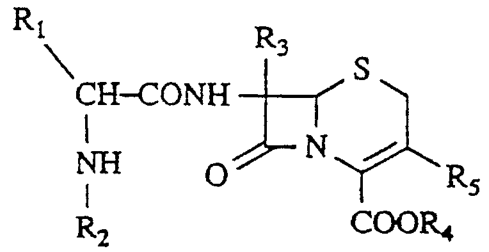

(I)

in which

$R_5$ represents hydrogen, halogen, azido or straight-chain, branched or cyclic, saturated or unsaturated alkyl having up to 6 C atoms, which is optionally substituted by halogen, $OCONH_2$, $C_1$-$C_5$-alkoxy, $C_2$-$C_7$-acyloxy or $C_1$-$C_5$-alkylthio,

$R_1$ represents a radical of the formula

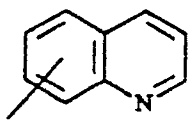 , 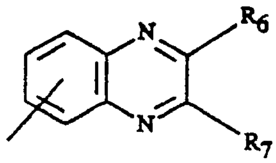 , 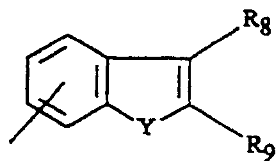

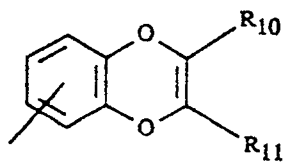 , 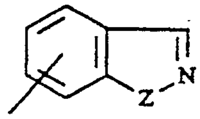 , 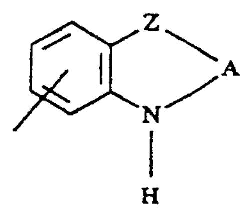

in which

$R_6$ and $R_7$ are identical or different and represent hydrogen, $C_6$-$C_{10}$-aryl, an amino group or hydroxyl,

$R_8$ and $R_9$ are identical or different and represent hydrogen, hydroxyl, an amino group,

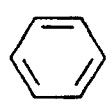 , 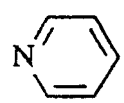 ,

alkyl having up to 8 C atoms, which is substituted by phenyloxy,

phenyl which is substituted by chlorine or amino, or

alkyl having up to 12 C atoms or

$R_8$ and $R_9$ together represent the group

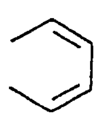

A represents the group

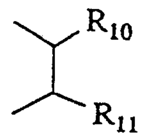

or

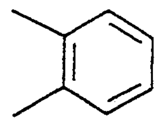

$R_{10}$ and $R_{11}$ are identical or different and represent hydrogen or alkyl having up to 12 C atoms,
Y represents oxygen or -NH,
Z represents oxygen, sulphur or -NH,
and in which
$R_2$ represents hydrogen or one of the following amino protecting groups,
tert.-butoxycarbonyl (Boc), carbobenzoxy (Cbz), 2-methoxycarbonyl-1-methylvinyl, trityl (Trt), benzyl (Bzl), benzyloxycarbonyl (Z), formyl or chloroacetyl,
$R_3$ represents hydrogen, alkoxy or alkylthio in each case having up to 5 C atoms, and
$R_4$ represents hydrogen or one of the following carboxyl protecting groups
tert.-butyl, decyl, 2,2,2-trichlorethyl, benzyl, 4-methoxybenzyl, 4-nitrobenzyl, triphenylmethyl, diphenylmethyl, acetoxymethyl, pivaloyloxymethyl, allyl or trimethylsilyl,
or alkali metal ions or ammonium ions or a radical of the formula $-CH_2-O-CO-C(CH_3)_3$ or $-CH-(CH_3)-O-CO-O-C_2H_5$, characterized in that compounds of the general formula (IIa)

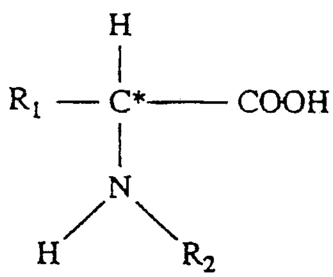

(IIa)

in which
$R_1$ has the meaning indicated above and in which
$R_2$ represents an amino protecting group customary in $\beta$-lactam chemistry,
are reacted, after activation of the carboxyl group by conversion into a mixed anhydride, after conversion into the mesylate or after conversion into an activated ester, with compounds of the general formula (III)

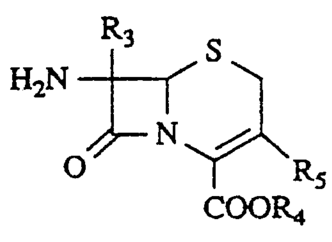

(III)

in which
$R_3$ and $R_4$ have the meaning indicated above, then, if desired, protecting groups are removed and the desired salts are prepared, or, from the salts, the free acids.

2. Process for the preparation of $\beta$-lactam compounds according to Claim 1, characterized in that
$R_5$ represents fluorine, chlorine, bromine, methoxy, methylthio, trifluoromethyl, methoxymethyl or methyl which is optionally substituted by halogen or $OCONH_2$, vinyl, $-CH=CH-CH_3$ or $-CH=CH-C_2H_5$.

3. $\beta$-Lactam compounds of the general formula (I) according to Claim 1 for use in the control of diseases of the human and animal body.

4. Medicaments containing $\beta$-lactam compounds of the general formula (I) according to Claim 1.

5. Use of $\beta$-lactam compounds of the general formula (I) according to Claim 1 for the production of medicaments.

## Revendications

1. $\beta$-lactames de formule générale I

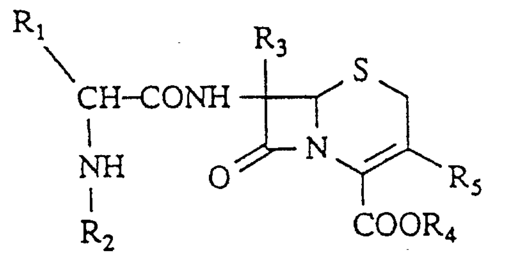

(I)

dans laquelle
$R_5$ représente l'hydrogène, un halogène, un groupe azido ou un groupe alkyle saturé ou insaturé, à chaîne droite, ramifiée ou cyclique, contenant jusqu'à 6 atomes de carbone, et qui est éventuellement substitué par des halogènes, des groupes $OCONH_2$, alcoxy en $C_1$-$C_5$, acyloxy en $C_2$-$C_7$ ou alkylthio en $C_1$-$C_5$,
$R_1$ représente un groupe de formules

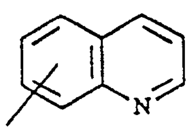
,
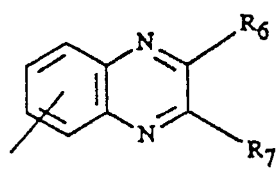
,
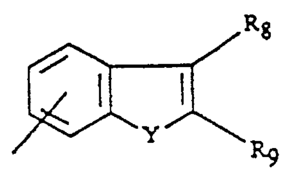

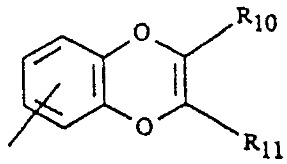
,
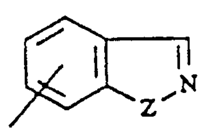
,
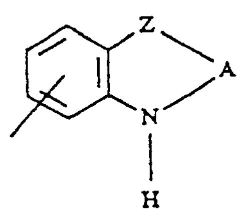

dans lesquelles
$R_6$, $R_7$, ayant des significations identiques ou différentes, représentent chacun l'hydrogène, un groupe aryle en $C_6$-$C_{10}$, un groupe amino ou hydroxy,
$R_8$ $R_9$, ayant des significations identiques ou différentes, représentent chacun l'hydrogène, un groupe hydroxy, un groupe amino,

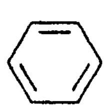
,
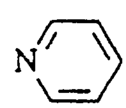
,

un groupe alkyle contenant jusqu'à 8 atomes de carbone et qui est substitué par un groupe phényloxy,

un groupe phényle qui est substitué par le chlore ou un groupe amino, ou un groupe alkyle contenant jusqu'à 12 atomes de carbone, ou bien $R_8$ et $R_9$ forment ensemble le groupe

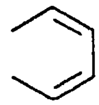 ,

A représente un groupe

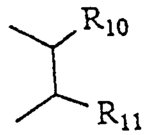 ou 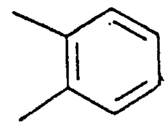 ,

$R_{10}$ $R_{11}$, ayant des significations identiques ou différentes, représentent chacun l'hydrogène, un groupe alkyle contenant jusqu'à 12 atomes de carbone,
Y représente l'oxygène ou -NH,
Z représente l'oxygène, le soufre ou -NH,
$R_2$ représente l'hydrogène ou l'un des groupes protecteurs suivants du groupe amino : tert-butoxycarbonyle (Boc), carbobenzoxy (Cbz), 2-méthoxycarbonyl-1-méthylvinyle, trityle (Trt), benzyle (Bzl), benzyloxycarbonyle (Z), formyle ou chloracétyle,
$R_3$ représente l'hydrogène, un groupe alcoxy ou alkylthio contenant chacun jusqu'à 5 atomes de carbone, et
$R_4$ représente l'hydrogène ou l'un des groupes protecteurs suivants du groupe carboxy : tert-butyle, décyle, 2,2,2-trichloréthyle, benzyle, 4-méthoxybenzyle, 4-nitrobenzyle, triphénylméthyle, diphénylméthyle, acétoxyméthyle, pivaloyloxyméthyle, allyle ou triméthylsilyle,
des ions alcalins ou d'ammonium ou un groupe de formule $-CH_2-O-CO-C(CH_3)_3$ ou $-CH(CH_3)-O-CO-O-C_2H_5$.

2. $\beta$-lactames selon la revendication 1, caractérisées en ce que :
$R_5$ représente le fluor, le chlore, le brome, un groupe méthoxy, méthylthio, trifluorométhyle, méthoxyméthyle, ou un groupe méthyle, vinyle, $-CH=CH-CH_3$, $-CH=CH-C_2H_5$ éventuellement substitué par des halogènes ou des groupes $OCONH_2$.

3. Procédé de préparation des $\beta$-lactames de la revendication 1, caractérisé en ce que l'on fait réagir des composés de formule générale IIa

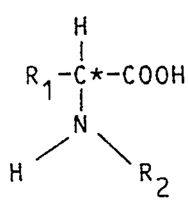

(IIa)

dans laquelle
$R_1$ a les significations indiquées dans la revendication 1, et
$R_2$ représente un groupe protecteur du groupe amino, usuel dans la chimie des $\beta$-lactames,
après activation du groupe carboxyle par conversion en un anhydride mixte, après conversion en le méthane-sulfonate ou après conversion en un ester activé, avec des composés de formule générale III

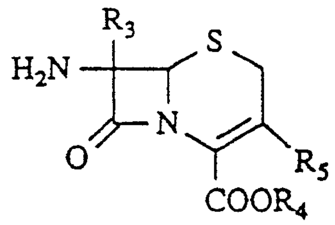

(III)

dans laquelle $R_3$ et $R_4$ ont les significations indiquées dans la revendication 1, puis, le cas échéant, on élimine les groupes protecteurs et on prépare les sels recherchés ou bien, à partir des sels, les acides libres.

4. $\beta$-lactames de forme générale I de la revendication 1, pour l'utilisation dans le traitement des maladies de l'organisme humain et animal.

5. Médicament contenant des $\beta$-lactames de formule générale I de la revendication 1.

6. Utilisation des $\beta$-lactames de formule générale I de la revendication 1 pour la préparation de médicaments.

Revendications pour l'Etat contractant suivant : AT

1. Procédé de préparation des $\beta$-lactames de formule générale I

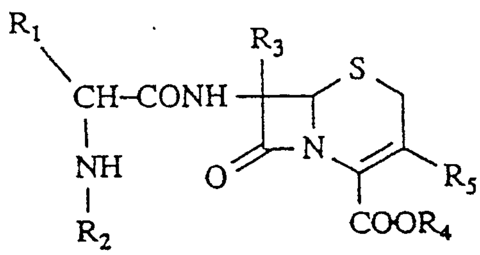

(I)

dans laquelle
$R_5$ représente l'hydrogène, un halogène, un groupe azido ou un groupe alkyle saturé ou insaturé à chaîne droite, ramifiée ou cyclique, contenant jusqu'à 6 atomes de carbone et éventuellement substitué par des halogènes, des groupes $OCONH_2$, alcoxy en $C_1$-$C_5$, acyloxy en $C_2$-$C_7$ ou alkylthio en $C_1$-$C_5$,
$R_1$ représente un groupe de formules

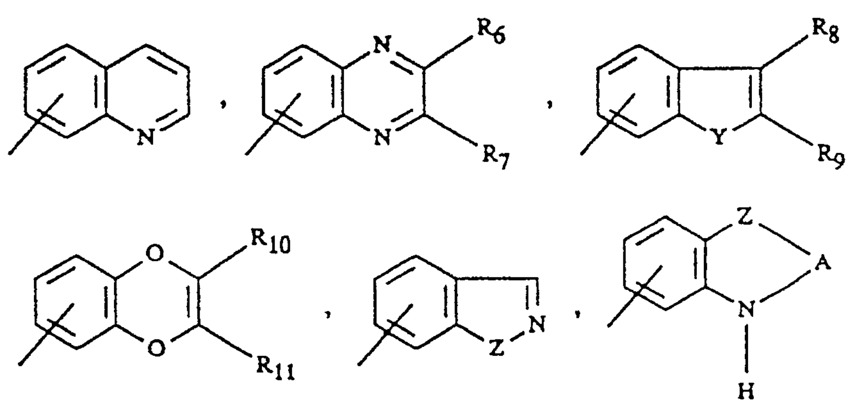

dans lesquelles
$R_6$, $R_7$, ayant des significations identiques ou différentes, représentent chacun l'hydrogène, un groupe

aryle en $C_6$-$C_{10}$, un groupe amino ou hydroxy,
$R_8$ $R_9$, ayant des significations identiques ou différentes, représentent chacun l'hydrogène, un groupe hydroxy ou amino,

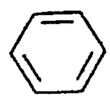 , 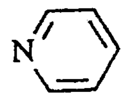 ,

un groupe alkyle contenant jusqu'à 6 atomes de carbone et substitué par un groupe phényloxy,
un groupe phényle substitué par le chlore ou un groupe amino,
ou un groupe alkyle contenant jusqu'à 12 atomes de carbone, ou bien
$R_8$ et $R_9$ forment ensemble le groupe

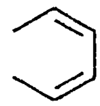 ,

A représente un groupe

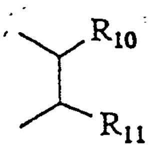 ou 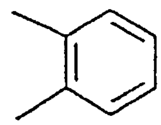 ,

$R_{10}$ $R_{11}$, ayant des significations identiques ou différentes, représentent chacun l'hydrogène ou un groupe alkyle contenant jusqu'à 12 atomes de carbone,
Y représente l'oxygène ou -NH,
Z représente l'oxygène, le soufre ou -HH,
$R_2$ représente l'hydrogène ou l'un des groupes protecteurs suivants du groupe amino : tert-butoxycarbonyle (Boc), carbobenzoxy (Cbz), 2-méthoxycarbonyl-1-méthylvinyle, trityle (Trt), benzyle (Bzl), benzyloxycarbonyle (Z), formyle ou chloracétyle,
$R_3$ représente l'hydrogène, un groupe alcoxy, alkylthio contenant chacun jusqu'à 5 atomes de carbone
$R_4$ représente l'hydrogène ou l'un des groupes protecteurs suivants du groupe carboxy : tert-butyle, décyle, 2,2,2-trichloréthyle, benzyle, 4-méthoxybenzyle, 4-nitrobenzyle, triphénylméthyle, diphénylméthyle, acétoxyméthyle, pivaloyloxyméthyle, allyle ou triméthylsilyle,
des ions alcalins ou des ions ammonium ou un groupe de formule $-CH_2-O-CO-C(CH_3)_3$ ou $-CH(CH_3)-O-CO-O-C_2H_5$,
caractérisé en ce que l'on fait réagir des composés de formule générale IIa

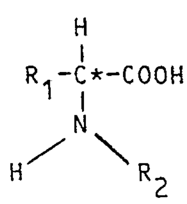 (IIa)

dans laquelle
$R_1$ a les significations indiquées ci-dessus, et
$R_2$ représente un groupe protecteur du groupe amino, usuel dans la chimie des $\beta$-lactames,
après activation du groupe carboxyle par conversion en un anhydride mixte, après conversion en le méthane-sulfonate ou après conversion en un ester activé, avec des composés de formule générale III

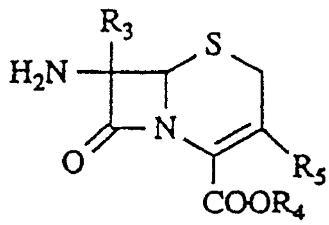

(III)

dans laquelle $R_3$ et $R_4$ ont les significations indiquées ci-dessus puis, le cas échéant, on élimine les groupes protecteurs et on prépare les sels recherchés ou bien, à partir des sels, les acides libres.

**2.** Procédé de préparation des $\beta$-lactames selon la revendication 1, caractérisé en ce que $R_5$ représente le fluor, le chlore, le brome, un groupe méthoxy, méthylthio, trifluorométhyle, méthoxyméthyle, ou un groupe méthyle, vinyle, $-CH=CN-CH_3$, $-CH=CH-C_2H_5$ éventuellement substitué par des halogènes ou des groupes $OCONH_2$.

**3.** $\beta$-lactames de formule générale I de la revendication 1, pour l'utilisation dans le traitement des maladies de l'organisme humain et animal.

**4.** Médicament contenant des $\beta$-lactames de formule générale I de la revendication 1.

**5.** Utilisation des $\beta$-lactames de formule générale I de la revendication 1 pour la préparation de médicaments.